# EUROPEAN PATENT SPECIFICATION

(11) **EP 1 997 491 B1**
(45) Date of publication and mention of the grant of the patent: **22.09.2010**
(21) Application number: 08162593.1
(22) Date of filing: 14.07.2005
(51) Int. Cl.: A61K 31/41, A61K 31/47, A61K 31/405, G01N 33/50

(54) **Method for inhibiting tumor metastasis**
Verfahren zur Hemmung von Tumormetastasierung
Procédé d'inhibition d'une métastase de tumeur

(30) Priority: 14.07.2004 US 587507 P
(43) Date of publication of application: 03.12.2008
(62) Divisional of application: 05771080.8
(73) Proprietor: Inflammation Research Center Company Ltd., Minami-ku, Sapporo Hokkaido 005-0832 (JP)
(72) Inventor: Nozaki, Masako, Sapporo Hokkaido 320-0508 (JP)
(74) Representative: Wittop Koning, Tom Hugo

(56) References cited:
- EP-A- 1 424 101
- WO-A-00/07977
- WO-A-00/53198
- WO-A-02/34715
- WO-A-02/34761
- WO-A-96/02496
- WO-A-96/13491
- WO-A-96/13500
- WO-A-97/21678
- US-B1- 6 492 332
- OHTSUKA YOSHIKAZU ET AL: "Pranlukast regulates tumour growth by attenuating IL-4 production in Kimura disease" EUROPEAN JOURNAL OF PEDIATRICS, vol. 163, no. 7, 16 April 2004 (2004-04-16), pages 416-417, XP002500356
- CHEN X ET AL: "LEUKOTRIENE A4 HYDOLASE AS A TARGET FOR CANCER PREVENTION AND THERAPY" CURRENT CANCER DRUG TARGETS, vol. 4, no. 3, 1 May 2004 (2004-05-01), pages 267-283, XP009043805
- GUNNING WILLIAM T ET AL: "Chemoprevention by lipoxygenase and leukotriene pathway inhibitors of vinyl carbamate-induced lung tumors in mice" CANCER RESEARCH, vol. 62, no. 15, 2002, pages 4199-4201, XP002500357
- STEELE V E ET AL: "LIPOXYGENASE INHIBITORS AS POTENTIAL CANCER CHEMOPREVENTIVES" CANCER EPIDEMIOLOGY, BIOMARKERS AND PREVENTION, vol. 8, no. 5, 1999, pages 467-483, XP000984604
- LEE K S ET AL: "Cysteinyl leukotriene receptor antagonist regulates vascular permeability by reducing vascular endothelial growth factor expression" JOURNAL OF ALLERGY AND CLINICAL IMMUNOLOGY, vol. 114, no. 5, 1 November 2004 (2004-11-01), pages 1093-1099, XP004631207

## Description

### BACKGROUND OF THE INVENTION

### Field of the Invention

The present invention relates to methods for inhibiting tumor metastasis and for inhibiting tumor cell adhesion to endothelial cells and/or capillary permeability in the form of transendothelial migration of tumor cells.

### Description of the Related Art

Arachidonic acid (AA) is released from phospholipid in the cell membrane to the cytoplasm in response to a number of insults such as mechanical, thermal, chemical, bacterial and other insults, and its products (the eicosanoid compounds prostaglandins and leukotrienes) have been found to be biologically important in a number of ways. Most of the eiconsanoid compounds tend to aggravate inflammatory, pain, and fever responses, and they have been the targets of extensive research on anti-inflammatory and analgesic drugs. For example, anti-inflammatory steroids such as cortisone function by suppressing the phospholipase enzymes that generate arachidonic acid from membrane phospholipids. Pain-killers such as aspirin and ibuprofen act by blocking to some extent the cyclooxygenase enzymes that control the conversion of arachidonic acid to the eicosanoids, prostaglandins, prostacyclins, and thromboxanes.

Additionally, it is known that prostaglandins and leukotrienes contribute to the genesis of inflammation in both the peripheral and central nervous system (CNS). Despite studies done over three decades, exactly how prostaglandins contribute to inflammation remains unclear. In contrast, recent studies using leukotriene receptor antagonists indicate that leukotrienes might play a major role in this process.

Leukotrienes are potent lipid mediators and are divided into two classes, based on the presence or absence of a cysteinyl group. Leukotriene B₄ does not contain such a group, whereas leukotriene C₄, D₄, E₄ and F₄ are cysteinyl leukotrienes. These compounds have been recognized as inflammatory agents since the early 1980's (von Sprecher et al., 1993 and Piper, 1984).

In the 1990's, various drugs known as "leukotriene antagonists", which can suppress and inhibit the activity of leukotrienes in the body, were identified. The term "leukotriene antagonist" is used herein in the conventional medical sense, to refer to a drug that suppresses, blocks, or otherwise reduces or opposes the concentration, activity, or effect of one or more subtypes of naturally occurring leukotrienes. However, such leukotriene antagonists can be classified into two different groups based on a difference in mechanism of action, one which to suppresses 5-lipoxygenase and the other which competitively antagonizes the receptor for leukotriene. Pranlukast, which is one leukotriene antagonist, acts strictly at the leukotriene C4 and D4 receptor level.

Although some leukotriene receptor antagonists have been disclosed for use in treating brain inflammation (e.g., J63258-879-A, JO2-169583-A, WO9959964-A1 EP-287-471-A), all of the disclosed leukotriene receptor antagonists are presumed to pass the blood-brain barrier (BBB) because it is conventional wisdom that a molecule must be able to pass the blood-brain barrier in order to reduce or inhibit brain inflammation, or to treat disorders of the brain resulting from brain inflammation (Wilkinson et al., 2001). As leukotriene C₄ and D₄ receptor antagonists do not pass the blood-brain barrier, insofar as is known, the leukotriene C₄ and D₄ receptor antagonists have never previously been used to treat or prevent brain inflammation except for a neuroprotective effect of pranlukast (ONO-1078), a leukotriene receptor antagonist, on focal cerebral ischemia in rats (Zhang et al., 2002) and in mice (Zeng et al., 2001). The leukotriene C₄ and D₄ receptor antagonists are, however, commonly used to treat asthma. The leukotriene C₄ and D₄ receptor antagonist pranlukast is used clinically as an anti-asthmatic drug and is known to have few side effects. Pranlukast does not pass, or passes the blood-brain barrier at most at a very minimal level, akin to the other antagonists such as zafirlukast and montelukast. Other off-label uses have been suggested for these compounds, including treatment of allergic diseases (Shih, U.S. Patent No. 6,221,880) and for use in treating migraine and cluster headaches (Sheftell et al., U.S. Patent No. 6,194,432). Demopulos et al., U.S. Patent No. 6,492,332, also reported the use of a leukotriene receptor antagonist as an anti-inflammatory in a method and composition for inhibiting tumor cell adhesion, pain and inflammation where a combination of agents, including another agent which inhibits tumor cell adhesion/attachment, and/or invasion and/or local metastasis, are administered to a patient undergoing a surgical procedure to remove a tumor.

The migration of leukocytes to sites of acute or chronic inflammation involves adhesive interactions between these cells and the endothelium. This specific adhesion is the initial event in the cascade that is initiated by inflammatory insults, and it is, therefore, of paramount importance to the regulated defense of the organism. The types of cell adhesion molecules that are involved in the interaction between leukocytes and the endothelium during an inflammatory response currently stands at four: (1) selectins; (2) (carbohydrate and glycoprotein) ligands for selectins; (3) integrins; and (4) integrin ligands, which are members of the immunoglobulin gene superfamily.

Selectins are cell-surface carbohydrate binding proteins that mediate cell adhesion between leukocytes and the vascular endothelial surface. For example, binding of e-selectin to its ligand expressed on the surface of circulating neutrophils initiates rolling, an early step in the recruitment of these cells to a site of injury or inflammation.

Cancer is a leading cause of death in developed countries. Metastasis, the spread of cells from a primary neoplasm to distant sites and their growth there is a most fearsome aspect of cancer. This fear is well founded. Despite significant improvements in early diagnosis, surgical techniques, general patient care, and local and systemic adjuvant therapies, most deaths from cancer are due to metastasis that are resistant to conventional therapies. Growing evidence suggests that carbohydrate-mediated cancer/tumor cell adhesion to selectins on the vascular endothelium is involved in the metastasis of a wide variety of epithelial cancers, including gastric, colorectal, pancreatic, liver, ovary, head and neck, and breast cancers, etc.

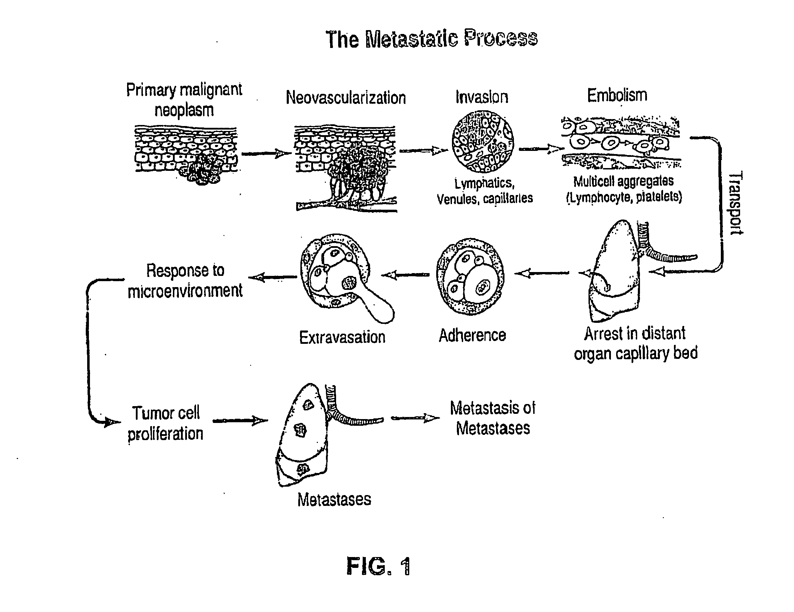

The metastatic process is illustrated in Fig. 1, where:

1) After the initial transforming event, either unicellular or multicellular growth of neoplastic cell must be progressive.

2) Extensive vascularization must occur if a tumor mass is to exceed 2mm in diameter.

3) The synthesis and secretion of several angiogenesis factors play a key role in establishing a neocapillary network from surrounding host tissue.

4) Local invasion of the host stroma by some tumor cells could occur by several mechanisms that are not mutually exclusive. Thin-wall venule-like lymphatic channels offer very little resistance to penetration by tumor cells and provide the most common pathways for tumor cells entry into the circulation.

5) Detachment and embolization of small tumor cell aggregates occur.

6) Tumor cells that survive the circulation must arrest in the capillary beds of organs. E- and p-selectins appear on activated endothelial cells to interact with tumor cells through sialyl-Lewis^{x} and sialyl-Lewis^{a} antigens.

7) Exravasation occurs and

8) Proliferation within the organ parenchyma completes the metastatic process.

Adhesion of malignant cells to the vascular endothelial surface involves a family of adhesion receptors similar to those involved in the recruitment of inflammatory cells to tissue sites. Selectins are involved in a cascade of sequential molecular steps following endothelial cell (EC) activation.

The selectins are cell adhesion molecules that are unified both structurally and functionally. Structurally, selectins are characterized by the inclusion of a domain with homology to a calcium-dependent lectin (C-lectins), an epidermal growth factor (egf)-like domain and several complement binding-like domains (Bevilacqua et al., 1989; Johnston et al., 1989; Lasky et al, 1989; Siegalman, et al., 1989; and Stoolman, 1989). Functionally, selectins share the common ability to mediate cell binding through interactions between their lectin domains and cell surface carbohydrate ligands (Brandley, et al., 1990; Springer and Lasky, 1991; Bevilacqua and Nelson, 1993 and Tedder et al., 1989).

There are three members identified so far in the selectin family of cell adhesion molecules: L-selectin (also called peripheral lymph node homing receptor (pnHR), LEC-CAM-1, LAM-1, gp90MEL, gp100MEL, gp110MEL, MEL-14 antigen, Leu-8 antigen, TQ-1 antigen, DREG antigen), E-selectin (LEC-CAM-2, LECAM-2, ELAM-1) and P-selectin (LEC-CAM-3, LECAM-3, GMP-140, PADGEM).

The carbohydrate determinants, sialyl Lewis^{a} (SLe^{a}) and sialyl Lewis^{x} (SLe^{x}), which are frequently expressed on human cancer cells, serve as ligands for e-selectin, which is expressed on vascular endothelial cells. These carbohydrate determinants are involved in the adhesion of cancer cells to vascular endothelium and thus contribute to metastasis of cancer. The selectins are inducible endothelial-expressed adhesion molecules involved in leukocyte recruitment. The initial adhesion mediated by these molecules triggers activation of integrin molecules through the action of several cytokines. The degree of expression of the carbohydrate ligands at the surface of cancer cells is well correlated with the frequency of metastasis. Monoclonal antibodies directed to Sle^{a} or Sle^{x} blocked adhesion of tumor cells (leukemia, colon carcinoma, and histiocytic lymphoma cells) to EC and platelets. Also selectin expression is frequently up-regulated on breast carcinoma endothelium.

P-selectin, also known as GMP-140 or PADGEM, is a membrane glycoprotein located in secretory granules of resting (unstimulated) platelets and endothelium. When mediators activate these cells, p-selectin is rapidly redistributed to the plasma membrane. The selectins constitute a family of structurally and functionally related molecules. Structural motifs common to each of these molecules include an N-terminal lectin-like domain followed by an EGF-like region, a series of consensus repeats related to those in complement-binding proteins, a transmembrane domain, and a short cytoplasmic tail. P-selectin is the receptor for neutrophils and monocytes when it is expressed on activated platelets and endothelium. This property facilitates rapid adhesion of leukocytes to endothelium at regions of tissue injury as well as platelet-leukocyte interactions at sites of inflammation and hemorrhage. Studies also have found that p-selectin binds to tumor cells in a variety of tissue sections and that it binds to the cell surface of a number of cell lines derived from carcinomas. Acting in concert with other adhesion molecules, p-selectin participates in tumor metastasis and is thus a target for drugs that block adhesion receptor function.

Studies supporting a role for selectins in metastasis are based upon the adhesion of human colon carcinoma cell lines to selectins using an *in vitro* flow model. Recombinant forms of p-selectin and Chinese hamster ovary cells expressing p-selectin supported attachment and rolling of KM12-L4 colon carcinoma cells and this effect was abolished by pretreatment of the KM12-L4 cells with neuraminidase. KM12-L4 cells interact with p-selectin through a PSGL-1-independent adhesion pathway. An e-selectin-IgG chimera was also found to support sialylated moiety-dependent adhesion of colon carcinoma cells under fluid flow. Thus, sialylated moieties are involved in the selectin mediated adhesion of human cancer cells to IL-1-simulated endothelium under flow conditions. Furthermore, the efficiency of e-selectin-mediated binding of human colon carcinoma cells to human and mouse EC has been found to be correlated with the metastatic potential of the cancer cells.

Recent studies evaluated the role of e-selectin-sialyl Lewis^{x} (Sle^{x})/sialyl LewiS^{a} (Sle^{a}) interaction in mediating *in vitro* adhesion of two human colon cancer cell lines, HT-29 and COLO 201, to human umbilical cord endothelial cells (HUVEC). Colon cancer cell lines had a strong expression of carbohydrate epitopes and it was established that adhesion of HT-29 and COLO 201 cells to IL-1-stimulated HUVEC could be inhibited by a monoclonal antibody directed against e-selectin. Prior incubation of cells with two different antibodies directed against Sle^{x} and antibodies directed against related Lewis epitopes, Le^{x} and Le^{a}, had no significant effect on adhesion. Three antibodies directed against Sle^{a} differed in their capacity to inhibit the adhesion of HT-29 and COLO 201 cells. Thus, the Sle^{a} epitope is important for adhesion of colon cancer cells mediated predominantly by e-selectin.

Additional studies have demonstrated that pretreatment of HUVEC with tumor necrosis factor-α (TNF-alpha) augmented the binding of the COLO 205 carcinoma cell line. The increased adherence was both concentration-and time-dependent with maximal tumor cell attachment found at 4 hr. This result was consistent with increased expression of the adhesion molecule e-selectin on the endothelium. Incubation of TNF-stimulated HUVECs with BB11, an anti-e-selectin mAb, prior to the addition of COLO 205 resulted in complete inhibition of adherence of the tumor cells. These studies confirm the utility of specific antibodies directed against e-selectins to inhibit tumor cell attachment to endothelial cell targets. Other studies have found that pretreatment with either an anti-p-selectin or an anti-L-selectin monoclonal antibody (i.e., MAb PB 1.3 and MAb DREG-200), or a sialyl Lewis^{x}-containing oligosaccharide (Sle^{x}-OS) are effective in inhibiting cell adhesion.

Similar *in vitro* and *in vivo* studies have established a role for selectins in the process of metastasis using SW1990 cells derived from a human pancreatic malignancy. SW1990 cells also strongly express Sle^{a} and SLe^{x} antigens, CD44H, and β1 integrin. These cells exhibit binding activity to IL-1-activated HUVECS and human peritoneal mesothelial cells. The adhesion leading to implantation of cancer cells to endothelial cells was inhibited by treatment with the antibodies against Sle^{a} and against β1 integrin. In animal studies, treatments with the antibodies against Sle^{a} and β1 integrin each inhibited the development of liver metastasis in nude mice with SW1990 cells and prolonged their survival.

Y. Ohtsuka et al Eur. J. Pediatr. (2004 163:416-417 discloses the use of pranlukast to regulate tumour growth. WO0234761 discloses the use of leukotriene B4 receptor antagonists to treat metastasis.

Citation of any document herein is not intended as an admission that such document is pertinent prior art, or considered material to the patentability of any claim of the present application. Any statement as to content or a date of any document is based on the information available to applicant at the time of filing and does not constitute an admission as to the correctness of such a statement.

### SUMMARY OF THE INVENTION

The present invention relates to the use of leukotriene C4 and D4 receptor antagonists for the manufacture of a medicament for inhibiting tumour metastasis.

The present invention also relates to leukotriene C4 and D4 receptor antagonists for use in inhibiting tumour metastasis.

### BRIEF DESCRIPTION OF THE DRAWINGS

Figure 1 is a schematic illustration of the metastatic process.

Figure 2 is a graph depicting the action of ONO-1078 (pranlukast) on dextran-induced edema.

Figure 3 is a schematic of the position of the cannula when measuring changes in the CSF of rats during the sepsis experiments.

Figure 4A and 4B are graphs showing that the administration of arachidonic acid (3.25 µg/2 µl) causes inflammation in the central nervous system of two different individuals. Fig. 4A is rat #1 and Fig. 4B is rat #2.

Figure 5 is a graph showing the inhibitory effect of pranlukast (490 mg/kg, i.p.) on the inflammation caused by arachidonic acid (3.25 µg/2µm) in the rat central nervous system (CNS). The data was obtained from four rats.

Figure 6 shows the outside surface of a central capillary with its numerous ditches (shown by white arrows) between astrocites that are part of the blood brain barrier. These ditches open to increase capillary permeability and play an important role in extravasation of both white blood cells and cancer cells.

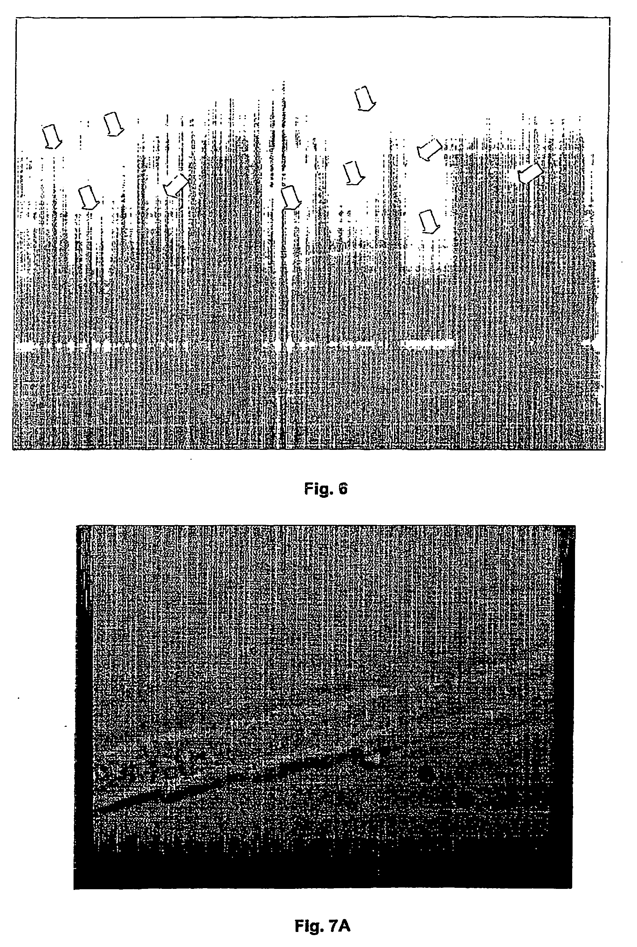

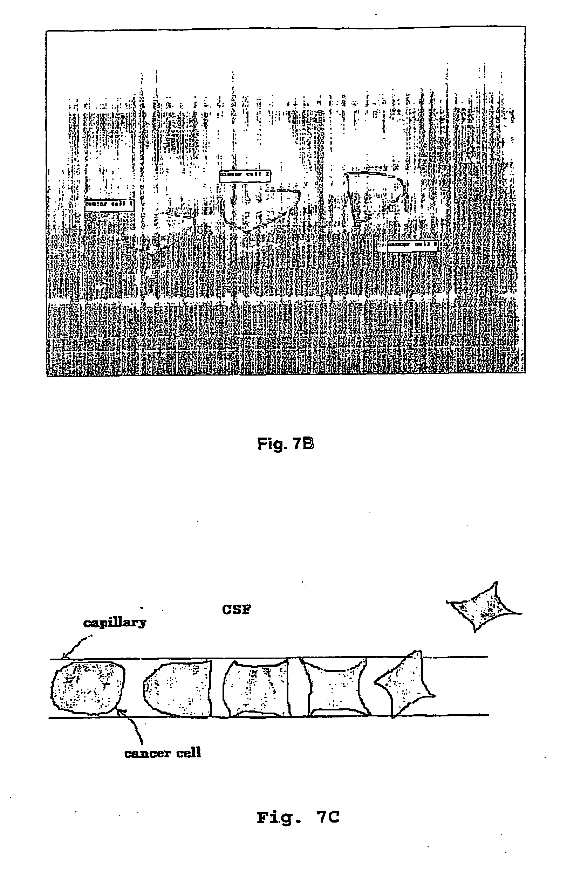

Figures 7A-7C show the change in shape of cancer cells during extravasation. Figs. 7A and 7B are the same except that in Fig. 7B, some of the cancer cells, which are stained with the fluorescent marker PKH67, are outlined by a drawn white line, e.g., cancer cells 1, 2 and 3. Cancer cell 1 and cancer cell 2 outlined in Fig. 7B are observed as parts of cells coming out from a central capillary into the CSF. Cancer cell 3 is just coming partway through the blood brain barrier to the CSF and its shape is not yet square. The black small squares that are visible are neutrophils that are adhering to p-selectin, integrin, and/or Ig-superfamily CAM (cell adhesion molecules) on endothelial cells. Transendothelial migration of cancer cells started immediately after arachidonic acid administration into the subarachnoid space. Transendothelial migration of white blood cells occurs later after arachidonic acid administration, being delayed compared to cancer cells. Fig. 7C schematically shows a sequence in which a cancer cell flattens in appearance so that it can pass through even the tiny narrow openings of the blood brain barrier.

Figure 8 is a time action graph of tumor or while blood cell (WBC) counts or CSF volume (µl) after administration of distilled water - PBS - saline to the animal using the experimental schedule in Table 1.

Figure 9 is a graph of a time action curve of WBC counts and CSF volume (µl) after administration of arachidonic acid (816 ng/2µl) into the subarachnoid space.

Figures 10A-10C are graphs of time action curves of tumor cell counts (Fig. 10A), CSF volume (Fig. 10B), and WBC counts (Fig. 10C) measured in the subarachnoid space after administration of distilled water - tumor cells (i.v.) - arachidonic acid (816 ng/2µl).

Figure 11 is a time action graph of tumor cell counts after administration of pranlukast (490 mg/kg) - PBS - arachidonic acid (816 ng/2µl).

Figures 12A-12C are graphs of area under time action curves for extravasation of tumor cells (Fig. 12A), for the effect of cancer cells on CSF volume (µl) caused by arachidonic acid (Fig. 12B), and for the effect of cancer cells on white blood cell counts caused by arachidonic acid (Fig. 12C). The asterisk represent statistical significance as analyzed by ANOVA with probability value p<0.05 to be signigicant.

### DETAILED DESCRIPTION OF THE INVENTION

The present disclosure provides a method for inhibiting tumor metastasis, involving administering an effective amount of a leukotriene C4 and D4 receptor antagonist, preferably pranlukast, or a pharmaceutically acceptable salt thereof, either to a subject in need thereof who is not concurrently undergoing a surgical procedure, such as for the removal of a tumor, or to a subject in need thereof in the absence of any other compounds having anti-tumor cell adhesion, anti-invasion or anti-metastasis properties, such as those compounds disclosed in U.S. Patent 6, 992, 332.
The present invention concerns the use of leukotriene C4 and D4 receptor antagonists for the manufacture of a medicament for inhibiting tumour metastasisaccording to claims 1-6.
The present invention also concerns leukotriene C4 and D4 receptor antagonists for use in inhibiting tumour metastasis according to claims 7-12.

The term "leukotriene antagonist" is used herein in the conventional medical sense to refer to a drug that suppresses, blocks, or otherwise reduces or opposes the concentration, activity, or effects of one or more subtypes of naturally occurring leukotrienes. A leukotriene antagonist typically antagonizes the actions of leukotriene at the receptor level and is more preferably referred to herein as a "leukotriene C4 and D4 receptor antagonist".

As used herein, the term "tumor" means a mass of transformed cells that are characterized, at least in part, by containing angiogenic vasculature. The term "tumor" is used broadly to include the tumor parenchymal cells as well as the supporting stroma, including the angiogenic blood vessels that infiltrate the tumor parenchymal cell mass. Although a tumor generally is a malignant tumor, i.e., a cancer having the ability to metastasize, a tumor also can be nonmalignant, provided that neovascularization is associated with the tumor. Tumor cells contemplated herein are derived form metastatic tumors. As used herein, the term "metastasis" refers to a secondary tumor that grows separately from the primary tumor and has arisen from detached, transported cells.

The present inventor had earlier studied the inflammation mechanism with the use of leukotriene C4 and D4 receptor antagonists, in particular, pranlukast. Generally, when an inflammatory response develops, various cytokines and other inflammatory mediators act upon the local blood vessels, and increase the expression of endothelial CAM. It is believed that these pro-inflammatory cytokines and inflammatory mediators activate p-selectin and e-selectin on the endothelial cells of the capillary and then start the process of white blood cell extravasation that sequentially follows the steps of (1) Rolling, (2) Triggering, (3) Arrest/Adhesion, and (4) Transendothelial Migration.

The process of inflammation is understood as follows:

1) Macrophages activated by nociceptive stimulus secret TNF-α, interleukin-1β and interleukin-6 in tissues.

2) Vascular endothelial cells increase the expression of e-selectin by TNF-α, and interleukin-1β, and p-selectin by histamine and thrombin in the capillaries.

3) Circulating white blood cells express mucins such as PSGL-1 or the tetrasaccharides sialyl Lewis and sialyl Lewis^{a} and sialyl Lewis^{x}, which bind to e- and p-selectin. This binding mediates the attachment or tethering of white blood cells to the vascular endothelium, allowing the cells to roll in the direction of the blood flow (Rolling). Although p-selectin and sialyl Lewis-dependent alterations are induced by leukotriene C₄/D₄ in the mid-jejunum of rats, the leukotriene receptor antagonist was not effective. Since then, potent selectin inhibitors have been developed.

4) As white blood cells roll, chemokines (interleukin 8, MIP-1α/β, and MCP-1) and PAF, the complement split (C5a,C3a and C5b67) and various N-formyl peptide are produced. Binding of these chemoattractants to receptors on the white blood cell membrane triggers an activating signal mediated by G-proteins associated with the receptor. This signal induces a conformational change in the integrin molecules in the white blood cell membrane, increasing their affinity for immunoglobulin-superfamily adhesion molecules on the endothelium (Triggering).

(5)Subsequent interaction between integrin and immunoglobulin-superfamily CAMs stabilizes adhesion of the white blood cell to endothelial cell, enabling the cell to adhere firmly to the endothelial cell(Arrest/adhesion).

The white blood cell then migrates through the vessel wall into the tissue. The steps in transendothelial migration and how it is directed are still largely unknown. They may be mediated by molecules binding between the surface of white blood cell and CD31 on the capillary endothelial cell, or by the binding LFA-1 on the white blood cells and JAM on the capillary endothelial cell (Transendothelial Migration).

Thus, white blood extravasation is a sequential reaction that is caused by interaction among capillary endothelial cells, molecules on the capillary endothelial cells, chemokines and adhesion molecules (selectin family, integrin family, and immunoglobulin-superfamily). However, it has not been previously known that leukotriene C₄ and D₄ play any role in the white blood cell extravasation in the central capillaries.

The leukotriene C₄ and D₄ receptor antagonist, pranlukast, was found by the present inventor to competitively antagonize the leukotriene C₄ and D₄ receptor and effectively inhibits brain inflammation (central inflammation) and sepsis (systemic inflammation).

The present inventor previously discovered that dextran-induced rat paw edema was inhibited by pranlukast in a dose-dependent fashion. At a dosage of about 490 mg/kg, administered intraperitoneally, pranlukast completely inhibited dextran-induced paw edema. This suggested that leukotriene C₄ and D₄ receptor antagonists act at the endothelial cells in the capillaries and inhibit the increased permeability of the capillaries which was induced by dextran.

The site of action of the leukotriene C₄ and D₄ receptor antagonist pranlukast is on the inside of the capillary lumen (involving the capillary endothelial cells). Because pranlukast either does not cross or only minimally crosses the blood brain barrier, the anti-inflammatory effect of pranlukast is due to the inhibition of (increased) capillary permeability and the inhibition of white blood cell extravasation in the capillary lumen that involve adhesion of white blood cells to capillary endothelial cells, and the transendothelial migration of white blood cells. Thus, pranlukast inhibits brain inflammation without crossing the blood brain barrier. In the case of a brain inflammation that has increased the permeability of the blood brain barrier, pranlukast in the plasma can pass though the blood brain barrier which has increased the permeability and be delivered to the inflamed region.

In the general capillaries, the site of action of pranlukast is basically the same as that in central capillaries i.e., pranlukast has an anti-inflammatory effect through inhibition of white blood cell extravasation in the capillary lumen, involving adhesion of white blood cells to capillary endothelial cells, and the transendothelial migration of white blood cells. However, it may be distributed more widely in the peripheral tissues because the structure of general capillaries has more openings such as intracellular cleft, pinocytosis, and fenestra, compared to the structure of central capillaries.

The mechanism of action of pranlukast, as demonstrated by the data presented below, stabilizes endothelial cells at the post-capillary venula both peripherally and centrally and inhibits the permeability at the first phase in the process of inflammation. Inflammatory responses occur in three distinct phases, each apparently mediated by different mechanisms:

1. An acute transient first phase - vasodilation and increased capillary permeability.

2. A delayed subacute second phase - infiltration of leukocytes and phagocytic cells (white blood cell extravasations); and

3. A chronic proliferative third phase - tissue degeneration and fibrosis.

In the delayed subacute second phase, there are four sequential overlapping steps: (1) rolling where e- and p-selectins appear on activated endothelial cells for interaction with leukocytes through sialyl-Lewis^{x} and sialyl-Lewis^{a} antigens; (2) activation; (3) arrest/adhesion; and (4) transendothelial migration. The e- and p-selectins can also interact with tumor cells in the process of metastasis as discussed in the "Description of the Related Art" section above. As the present inventor expects that there is a common mechanism between white blood cell extravasation in inflammation and tumor (cancer) cell extravasation in metastasis, leukotriene antagonists, preferably leukotriene C4 and D4 receptor antagonists, which inhibit white blood cell extravasation, can also be used to inhibit tumor cell extravasation in the process of metastasis.

A leukotriene C4 and D4 receptor antagonist and most preferably pranlukast, can be used in the present invention to inhibit tumor metastasis in a patient in need thereof who has a tumor or who is going to have a tumor surgically removed in the future. The present invention also inhibits tumor cell adhesion to endothelial cells, preferably capillary endothelial cells, and/or inhibits capillary permeability in the form of transendothelial migration of tumor cells by causing a leukotriene antagonist to contact the endothelial cells to thereby block tumor cell adhesion to endothelial cells contacted with the leukotriene antagonist and/or inhibit capillary permeability in the form of transendothelial migration of tumor cells.

The preferred leukotriene C4 and D4 receptor antagonist used in the present invention includes pranlukast (ONO-1078; Ono Pharmaceutical Company, Osaka, Japan), zafirlukast and montelukast or a pharmaceutically acceptable salt and/or hydrate thereof. The chemical structures of pranlukast, montelukast, and zafirlukast are shown below.

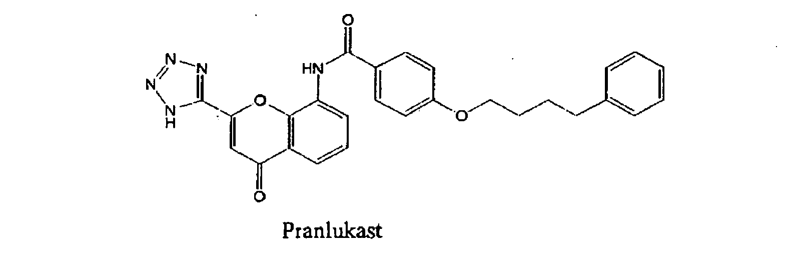

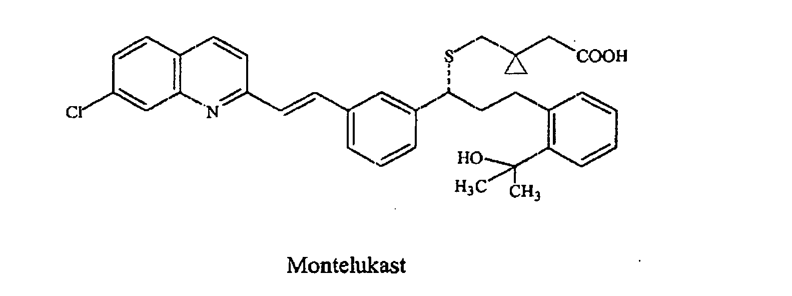

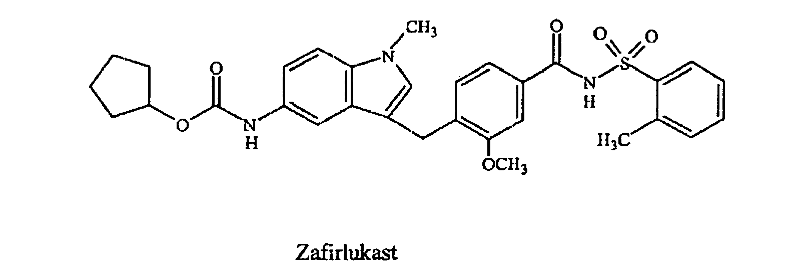

Pharmaceutically acceptable salts include alkaline metals such as lithium, sodium, potassium, etc., alkaline earth metals such as magnesium, calcium, etc., and aluminum. Non-limiting examples of other suitable leukotriene C4 and D4 receptor antagonists include the compounds, 8-[2-(E)-[4-(4-fluorophenyl)butyloxy]phenyl] vinyl]-4-oxo-2-[5-1H-tetrazolyl)-4H-1-benzopyran sodium salt (MEN-91507; Menarini), CR-3465 (Rottapharm), KP-496 (Kaken Pharmaceutical), 4-[6-acetyl-3-(3-[(4-acetyl-3-hydroxy-2-propylphenyl)thio] propoxy]-2-propylphenoxy]butanoic acid (MN-002; Kyorin Pharmaceutical; U.S. Patent 4,985,585), and (R)-3-methoxy-4-[1-methyl-5-[N-(2-methyl-4,4,4-trifluorobutyl)carbamoyl]indol-3-ylmethyl]-N-(2-methyl phenylsulfonyl)benzamide(MCC-847; Astra Zeneca; EP 531078).

While pranlukast, which is a leukotriene C4 and D4 receptor antagonist, is the most preferred leukotriene antagonist for use the present invention, a further preferred group of leukotriene C4 and D4 receptor antagonist compounds, similar in structure to pranlukast and having the properties of inhibiting tumor cell adhesion to endothelial cells and/or inhibiting capillary permeability to prevent transendothelial migration of tumor cells, are also included for use in the present invention. This preferred group of compounds are compounds having the general formula (I) 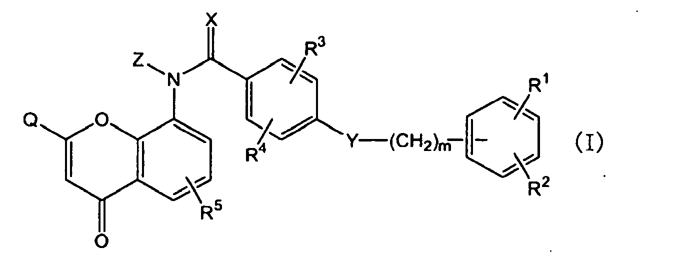 wherein:
R¹, R², R³, R⁴ and R⁵ are the same or different and is hydrogen, halogen, hydroxyl, amino, carboxy, nitro, cyano, lower alkyl, lower alkoxy, lower alkanoyl, lower alkanoyloxy, lower alkoxycarbonyl, mono or di lower alkyl substituted-amino, aralkyl, aryl, or heteroaryl;
Q is carboxyl, lower alkoxycarbonyl, or tetrazoyl;
X is oxygen, sulfur, or -NR- (wherein R is hydrogen or lower alkyl);
Z is hydrogen or lower alkyl; and
m is an integer from 1 to 6.

In the compound of formula (I), the compound where R¹, R², R³, R⁴, and R⁵ are hydrogen, Q is tetrazoyl, X and Y are oxygen, Z is hydrogen and m=4, is pranlukast as shown below.

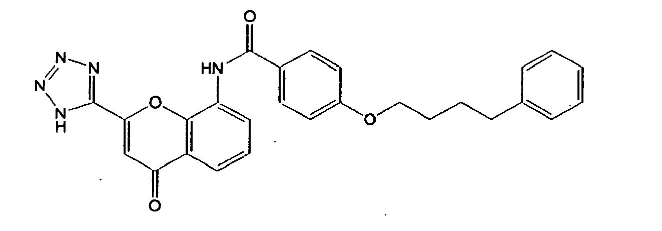

The substituent groups for the compound of formula (I) are further defined as follows:
A halogen is fluorine, chlorine, bromine, or iodine;
Suitable "lower alkyl" in the term "lower alkyl", "lower alkoxy", "lower alkoxycarbonyl", "mono or di lower alkyl substituted amino", may be straight or branched such as methyl, ethyl, propyl, isopropyl, butyl, isobutyl, tertbutyl, pentyl, hexyl, isohexyl, cyclopropyl, cyclobutyl, 2-methylcyclopropyl, cyclopropylmethyl, cyclopentyl, cyclohexyl, etc;
Suitable "lower alkanoyl" and "lower alkanoyloxy" in the term "lower alkanoyl" may be straight or branched or a loop of alkanoyloxy, for example, formyl, acetyl, propionyl, butyryl, isobutyryl, valeryl, isovaleryl, pivaloyl, hexanoyl, cyclopropylcarbonyl, cyclobutylcarbonyl,
   2-methylcyclooropylcarbonyl, cyclohexycarbonyl, etc;
   Aralkyl is an aralkyl radical of C₇-C₂₀, for example, benzyl, phenetyl, α-methlbenzyl, benzhydryl trityl, naphtylmethyl, etc; Aryl is an aryl radical of C₆-C₁₄, for example, phenyl and naphtyl; and
   Heteroaryl is a heteroaryl of C₃-C₈ and is single ring, many rings or combined rings containing the same or different 1-4 of N atom, O atom or S atom, for example, 2-pyridyl, 3-pyridyl, 4-pyridyl, 2-quinolyl, 3-quinolyl, 4-quinolyl, 5-quinolyl 6-quinolyl, 7-quinolyl, 8-quinolyl, 2-indolyl, 3-indolyl, 4-indolyl, 5 -indolyl, 6 -indolyl, 7-indolyl, 2-furil, 3-furil, 2-tieril,3-tieril, 2-pyrrolidyl, 3-pyrrolidyl, 2-imidazolyl, 4-imidazolyl, 5-imidazolyl , 2-oxazolyl, 4-oxazolyl, 5-oxazolyl,2-thiazolyl, 4-thiazolyl, 5-thiazolyl, etc.

Non-limiting examples of leukotriene antagonists/inhibitors beside the preferred leukotriene C4 and D4 receptor antagonists include tetrazole derivatives as leukotriene D4 and H1 antagonists (EP 0905133 A1), thiazolylbenzofuran derivatives (EP 0528337 A1), quinoline derivatives as leukotriene D4 antagonists, lipoxin A4 and derivatives as leukotriene D4 antagonists (U.S. Patent 5,079,261), echinacea as leukotriene antagonist (WO 99/21007), analogues of 16 hydroxyeicosatetraenoic acid (WO 99/59964), and antagonists that suppress 5-lipoxygenase.

The leukotriene C4 and D4 receptor antagonist pranlukast has been found to be safe (LD₅₀ > 2000 mg/kg (p.o.) and (s.c.) in both rats and mice). After single administration of pranlukast (30 mg/kg, 100 mg/kg, 300 mg/kg, 1000 mg/kg; p.o.), and repeated administration of pranlukast (30 mg/kg/day, 100 mg/kg/day, 300 mg/kg/day, 1000 mg/kg/day; p.o.) for three months and six months, rats showed normal behavior, changes in body weight, and food intake compared with those of the control group. The results of urine examination and histopathological examinations are also normal by the single administration and the repeated administration of pranlukast. The maximum blood concentration of pranlukast (administered at 20 mg/kg) was attained within one hour after the administration (p.o.) and maintained for at least 5 hours. However, no pranlukast was observed 24 hours after administration of such low doses of pranlukast (data from Ono Pharmaceutical Company).

As mentioned above, the anti-inflammatory effect of pranlukast inhibits the expression of p-selectin and/or e-selectin, leukocyte-specific cell adhesion molecules (CAMs) on endothelial cells or antagonizes leukotriene C₄ and D₄ receptors that play an important role during white blood cell extravasasions in the capillary lumen.

According to the present invention, the magnitude of an effective dose of a leukotriene C4 and D4 receptor antagonist for inhibiting tumor metastasis may vary with the severity of the condition to be treated and the route of administration, which route of administration is preferably oral. Accordingly, administration is most preferably systemic administration. The dose, and perhaps the dose frequency, will also vary according to the age, body weight, and response of the individual patient. In general, suitable oral daily dosage ranges of leukotriene antagonists can be readily determined by those of skill in the art. For example, see the Physician's Desk Reference, 54th Edition, Medical Economics Company Inc. (2000) for suitable dosages presently used for known leukotriene inhibitors for the treatment of asthma. For leukotriene C4 and D4 receptor antagonists, the oral daily dose range can be appropriately selected. Specifically for pranlukast, the acceptable dose is preferably 100 mg/day-2000 mg/day, more preferably 200 mg/day - 1000 mg/day, and most preferably 400 mg/day-800 mg/day. For montelukast, the dose range is preferably 5 mg/day - 100 mg/day, more preferably 5 mg/day - 50 mg/day, and most preferably 10 mg/day - 20 mg/day. For zafirlukast, the dose range is preferably 10 mg/day - 300 mg/day, more preferably 20 mg/day - 150 mg/day, and most preferably 40 mg/day - 80 mg/day.

It is further recommended that children, patients aged 65 years and older, and those with impaired renal or hepatic function initially receive low doses, and that they then be titrated up based on individual response(s) or the dose level in the blood. It may be necessary to use dosages outside these ranges in some cases, as will be apparent to those of skill in the art. Furthermore, it will be appreciated that the clinician or treating physician will know how and when to adjust, interrupt, or terminate therapy in conjunction with individual patient response.

The most common dosage form at present is an oral formulation for most presently available leukotriene antagonists because of the need to dissolve in stomach acid. Dosage forms may include tablets, troches, capsules, gel caps, lozenges, and the like. Due to their ease of administration, tablets and capsules represent the most advantageous oral dosage unit form, where solid pharmaceutical carriers are employed. In cases where the patient is unconscious, administration may preferably be by cannula to the stomach. If desired, tablets may be coated by standard aqueous or nonaqueous techniques. At least one presently available leukotriene C4 and D4 receptor antagonist, i.e., Singulair (montelukast), can be administered intravenously. Administration can be carried out once a day.

The present disclosure further provides a method of screening for an inhibitor of capillary permeability where tumor cells are inhibited from tranendothelial migration from capillary to cerebrospinal fluid. This screening method involves first administering a potential candidate inhibitor compound to a non-human mammal such as a rat, mouse, gerbil, dog, cat, rabbit, monkey, etc., followed shortly afterwards by administering tumor cells intravenously to the non-human mammal and later by administering an inflammation-inducing agent which causes an increase in capillary permeability into the subarachnoid space of the non-human mammal via a cannula inserted through the dura matter of the brain. After the administration of the inflammation-inducing agent, cerebrospinal fluid is collected and the counts of tumor cells that have extravasated from capillaries into the cerebrospinal fluid collected from the subarachnoid space are compared to a control with no administration of the potential inhibitor compound to determine if the potential inhibitor compound is an inhibitor of capillary permeability of tumor cells. This method optionally includes measuring the amount (volume) of cerebrospinal fluid collected, where the more cerebrospinal fluid volume collected, the more permeable the capillaries. The preferred non-human mammal is the rat.

Examples of the inflammation-inducing agent include arachidonic acid, prostaglandin, thromboxane, histamine, yeast, LPS, dextran, bradykinin, carrageenan, leukotriene, TNF-α, interleukin-1β or interleukin-6. Among them, a low molecular weight compound such as arachidonic acid is preferred.

The method for administering a potential candidate inhibitor compound to a non-human animal is not particularly limited and can be selected appropriately from known administration methods such as oral administration, subcutaneous administration, intra-peritoneal administration, intravenous administration, intramuscular administration, nasal (nasal drop) administration, inhalation, sublingual administration, suppository administration, etc., in view of, for example, chemical properties (such as lipid-solubility, etc.) of the potential candidate inhibitor compound. The assessment of whether a potential candidate inhibitor compound can act on endothelial cells covering the inside of the capillary lumen to inhibit the increased capillary permeability and the infiltration of tumor cells from the capillary to the tissue can be conducted by measuring tumor cell counts and/or an amount of collected cerebrospinal fluid. However, it is preferred that the tumor cell count in cerebrospinal fluid be measured together with the amount of cerebrospinal fluid. In addition, it is preferred that the cerebrospinal fluid is collected over time, e.g., every 30 minutes or one hour, and to assess the values relative to the controls (no administration of potential candidate inhibitor compound). Where the administration of the potential candidate inhibitor compound inhibits the exudation of cerebrospinal fluid and inhibits the presence of white blood cells in cerebrospinal fluid such as observed in the controls, then the potential candidate inhibitor compound can be regarded as an inhibitor of increased capillary permeability (particularly in the form of transendothelial migration of tumor cells) and is useful as a preventive agent for inhibiting increased capillary permeability when the inhibitor compound is administered prior to the introduction of an inflammation-inducing agent.

Having now generally described the invention, the same will be more readily understood through reference to the following examples which are provided by way of illustration and are not intended to be limiting of the present invention. Examples 1-4 are presented to show that the mode of action of the anti-inflammatory effect of a leukotriene C4 and D4 receptor antagonist is due to its ability to inhibit increase in capillary permeability and white blood cell extravasation in the capillary lumen, which includes capillary endothelial cells.

### Clauses

1. A method for inhibiting tumor metastasis, comprising administering to a subject in need thereof an effective amount of a leukotriene antagonist, or a pharmaceutically acceptable salt thereof, in the absence of any other compounds having anti-tumor cell adhesion, anti-invasion or anti-metastasis properties.
2. The method of clause 1, wherein the leukotriene antagonist, or a pharmaceutically acceptable salt thereof, is administered orally.
3. The method of clause 1, wherein the leukotriene antagonist is a leukotriene C4 and D4 receptor antagonist.
4. The method of clause 3, wherein the leukotriene C4
   and D4 receptor antagonist is a compound of formula (I) 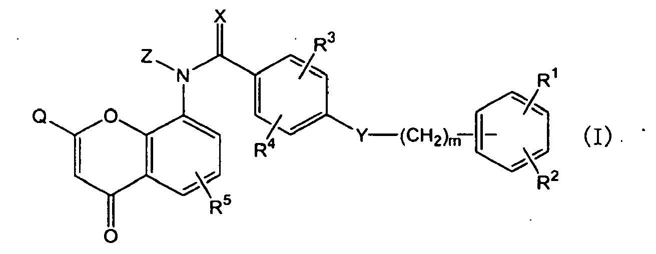 wherein:
   R¹, R², R³, R⁴ and R⁵ are the same or different and is hydrogen, halogen, hydroxyl, amino, carboxy, nitro, cyano, lower alkyl,
   lower alkoxy, lower alkanoyl, lower alkanoyloxy, lower alkoxycarbonyl, mono or di lower alkyl substituted-amino, aralkyl, aryl, or heteroaryl;
   Q is carboxyl, lower alkoxycarbonyl, or tetrazoyl;
   X is oxygen, sulfur, or -NR- (wherein R is hydrogen or lower alkyl);
   Z is hydrogen or lower alkyl; and
   m is an integer from 1 to 6.
5. The method of clause 4, wherein the leukotriene C4 and D4 receptor antagonist is pranlukast.
6. A method for inhibiting tumor metastasis, comprising administering an effective amount of a leukotriene antagonist, or a pharmaceutically acceptable salt thereof, to a subject in need thereof who is not concurrently undergoing a surgical procedure.
7. The method of clause 6, wherein the leukotriene antagonist, or a pharmaceutically acceptable salt thereof, is administered orally.
8. The method of clause 6, wherein the leukotriene antagonist is a leukotriene C4 and D4 receptor antagonist.
9. The method of clause 8, wherein the leukotriene C4
   and D4 receptor antagonist is a compound of formula (I) 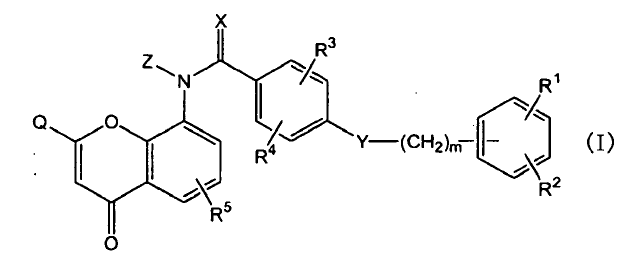 wherein:
   R¹, R², R³, R⁴ and R⁵ are the same or different and is hydrogen, halogen, hydroxyl, amino, carboxy, nitro, cyano, lower alkyl,
   lower alkoxy, lower alkanoyl, lower alkanoyloxy, lower alkoxycarbonyl, mono or di lower alkyl substituted-amino, aralkyl, aryl, or heteroaryl;
   Q is carboxyl, lower alkoxycarbonyl, or tetrazoyl;
   X is oxygen, sulfur, or -NR- (wherein R is hydrogen or lower alkyl);
   Z is hydrogen or lower alkyl; and
   m is an integer from 1 to 6.
10. The method of clause 9, wherein the leukotriene C4 and D4 receptor antagonist is pranlukast.
11. A method for inhibiting tumor cell adhesion to endothelial cells and/or inhibiting capillary permeability in the form of transendothelial migration of tumor cells, comprising causing an effective amount of a leukotriene antagonist, or pharmaceutically acceptable salt thereof, to contact endothelial cells to inhibit capillary permeability and/or tumor cell adhesion to the endothelial cells contacted with the leukotriene receptor antagonist.
12. The method of clause 7, wherein the leukotriene antagonist, or a pharmaceutically acceptable salt thereof, is administered orally.
13. The method of clause 11, wherein the leukotriene antagonist is a leukotriene C4 and D4 receptor antagonist.
14. The method of clause 13, wherein the leukotriene
   C4 and D4 receptor antagonist is a compound of formula (I) 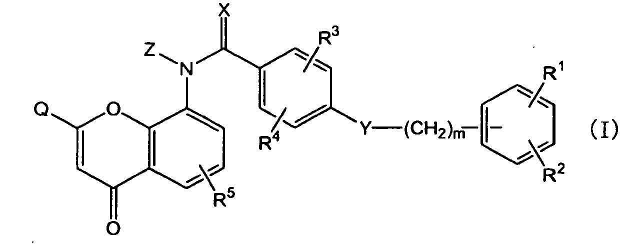 wherein:
   R¹, R², R³, R⁴ and R⁵ are the same or different and is hydrogen, halogen, hydroxyl, amino, carboxy, nitro, cyano, lower alkyl, lower alkoxy, lower alkanoyl, lower alkanoyloxy, lower alkoxycarbonyl, mono or di lower alkyl substituted-amino, aralkyl, aryl, or heteroaryl;
   Q is carboxyl, lower alkoxycarbonyl, or tetrazoyl;
   X is oxygen, sulfur, or -NR- (wherein R is hydrogen or lower alkyl);
   7. is hydrogen or lower alkyl; and
      m is an integer from 1 to 6.
15. The method of clause 14, wherein the leukotriene C4 and D4 receptor antagonist is pranlukast.
16. A method of screening for an inhibitor of capillary permeability to tumor cells, comprising:
   administering a potential candidate inhibitor compound to a non-human mammal;
   administering tumor cells intravenously to the non-human mammal just after administering the potential candidate inhibitor compound;
   administering an inflammation-inducing agent into the subarachnoid space of the non-human mammal via a cannula inserted therein through the dura mater of the brain, wherein the inflammation-inducing agent is an agent which causes an increase in capillary permeability;
   collecting cerebrospinal fluid; and
   determining from the collected cerebrospinal fluid counts of tumor cells which extravasated from capillaries, compared to the control without administration of the potential candidate inhibitor compound, if the potential candidate inhibitor compound is an inhibitor of capillary permeability to tumor cells.
17. The method of clause 16, wherein the inflammation-inducing agent is selected from the group consisting of arachidonic acid, prostaglandin, thromboxane, histamine, LPS, dextran, bradykinin, carrageenan, leukotriene, TNFα, IL-1β, and IL-6.
18. The method of clause 16, wherein the inflammation inducing agent is arachidonic acid.
19. The method of clause 16, further comprising measuring the amount of cerebrospinal fluid collected.
20. The method of clause 16, wherein the non-human mammal is a rat.
21. Use of a leukotriene antagonist, or a pharmaceutically-acceptable salt thereof, for the preparation or manufacture of a medicament for inhibiting tumor metastasis in the absence of any other compounds having anti-tumor cell adhesion, anti-invasion or anti-metastasis properties.
22. Use according to clause 21, wherein the leukotriene antagonist is a leukotriene C4 and D4 receptor antagonist.
23. Use according to clause 18, wherein the leukotriene C4 and D4 receptor antagonist is a compound of
   formula (I) 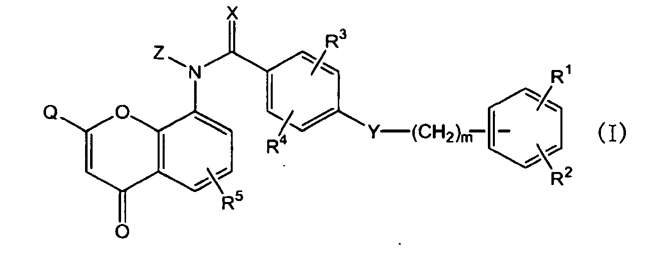 wherein:
   R¹, R², R³, R⁴ and R⁵ are the same or different and is hydrogen, halogen, hydroxyl, amino, carboxy, nitro, cyano, lower alkyl,
   lower alkoxy, lower alkanoyl, lower alkanoyloxy, lower alkoxycarbonyl, mono or di lower alkyl substituted-amino, aralkyl, aryl, or heteroaryl;
   Q is carboxyl, lower alkoxycarbonyl, or tetrazoyl;
   X is oxygen, sulfur, or -NR- (wherein R is hydrogen or lower alkyl);
   Z is hydrogen or lower alkyl; and
   m is an integer from 1 to 6.
24. Use according to clause 23, wherein the leukotriene C4 and D4 receptor antagonist is pranlukast.

### Example 1: Peripheral studies

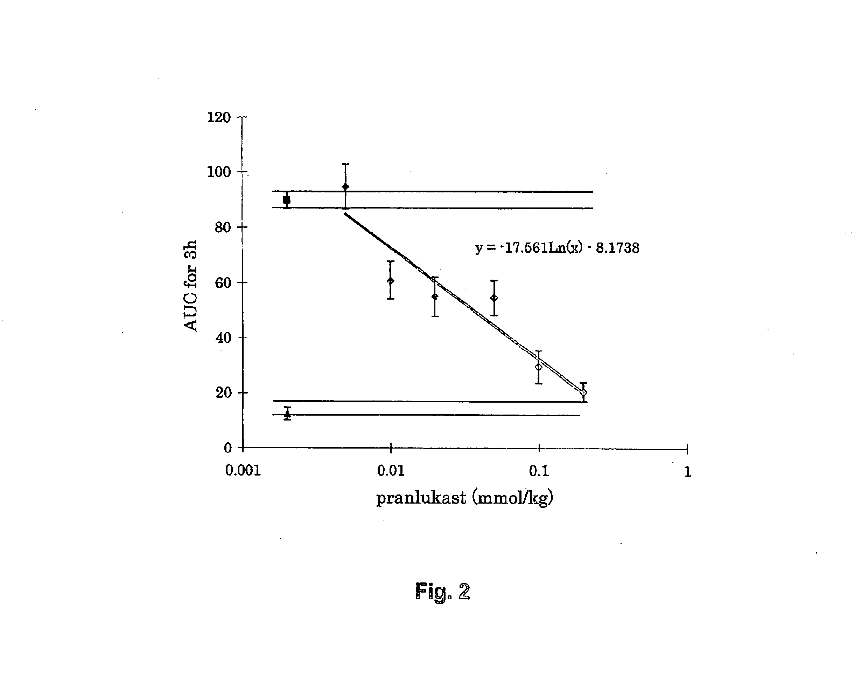

The present inventor discovered that dextran-induced rat paw edema was inhibited by pranlukast in a dose-dependent fashion. At a dosage of about 490 mg/kg, administered orally, pranlukast completely inhibited dextran-caused paw edema (Figure 2).

This suggested that LT antagonists might act at the endothelial cells in the peripheral capillary and inhibit the increased permeability of the capillary induced by dextran. In spite of the many openings such as clefts, fenestrae and pinocytic vesicles among endothelial cells in the capillary, pranlukast inhibited the permeability of the capillary. Since the endothelial cells in the brain capillary have less openings than other capillaries because of the presence of tight junctions, pranlukast may be more effective in inhibiting the permeability of the brain capillary than the permeability of a general capillary. Therefore, it is expected that such a mechanism might also come into play at the CNS level.

### Example 2: Measurement of Changes in the Inflammatory Process

To investigate the role of leukotriene antagonists in the treatment of inflammation, a sensitive and quantitative method to measure inflammation for the central nervous system was developed. Important changes in the inflammatory process can be monitored, as both the permeability of blood brain barrier (BBB) and the infiltration of white blood cells (WBC) to cerebrospinal fluid (CSF) caused by arachidonic acid can be measured over time from the same experimental animal. In particular, the effect of the leukotriene C₄ and D₄ antagonist, pranlukast, was studied using this method. Changes in the inflammatory process of both the permeability of BBB and the infiltration of white blood cells (WBC) caused by administering arachidonic acid as a nociceptive stimulus were observed.

### Materials and Methods

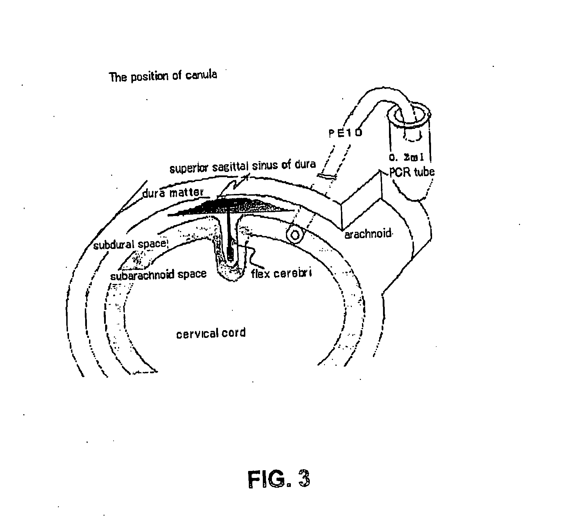

Male rats, approximately 350-400 g, were anesthetized with sodium pentobarbital at a dose of 50 mg/kg intraperitoneally (i.p.). Animals were then placed in a stereotaxic unit. Fur from the top of the head to neck was shaved. The.study area was disinfected with 70% ethanol followed by a 10% providone-iodine solution. Skin and muscle over the cervical cord were dissected until cervical vertebrae were exposed. A hole was made using a 23G3/4 needle passing through the dura via the cisterna magna. One end of a 2-3 mm PE10 cannula (0.011 "I.D., 0.007" thick, 0.024" O.D., 6.5 cm long) was inserted into the subarachnoid space at the cervical cord so as to avoid damage to neuronal tissue (Fig. 3). The cannula was fixed with cyanoacrylic glue to the peripheral tissue. The other end of PE10 was used for administration of a nociceptive stimulus, such as arachidonic acid (AA; 3.25 µg/2µl) and subsequently for collecting cerebrospinal fluid (CSF) every thirty minutes thereafter during the 11-hour experimental period. The cannula filled with CSF immediately after the cannulation.

Air was infused to inject AA to the subarachnoid space. The muscles and skin were closed with surgical staples. The rat was then removed from the stereotaxic unit and placed on a heat pad and continuously monitored.

In the interaction study, the leukotriene C4 and D4 antagonist, pranlukast, was administered (490 mg/kg, i.p.) thirty minutes before administration of arachidonic acid (AA). If the effects of the anesthesia appeared to be wearing off, an additional 0.05-0.1 ml pentobarbital (50 mg/ml, i.p.) dose was administered. If at any time during the experiment, the animal appeared to be in pain or distress, the study was immediately stopped and the animal was euthanized.

After the experiment, the animal was euthanized by exposure to high concentration of halothane gas in an enclosed jar in a chemical fume hood.

### Results

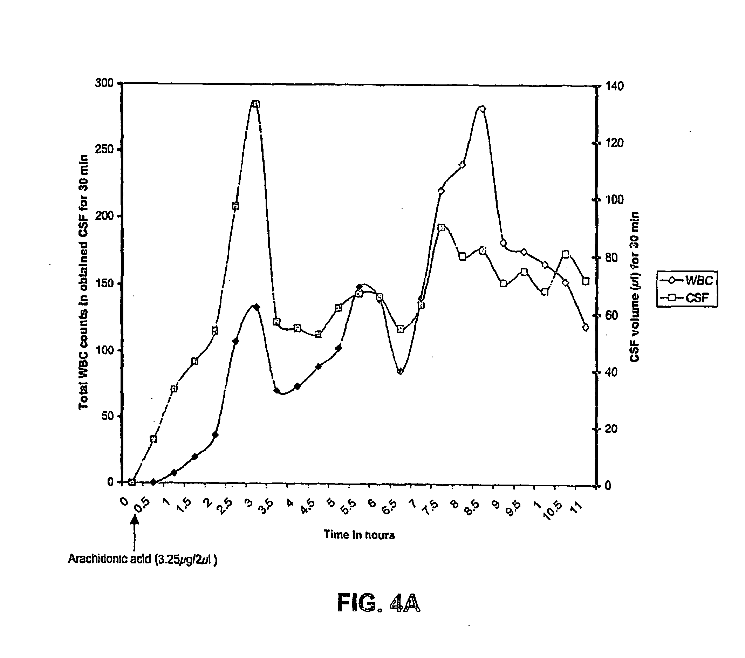

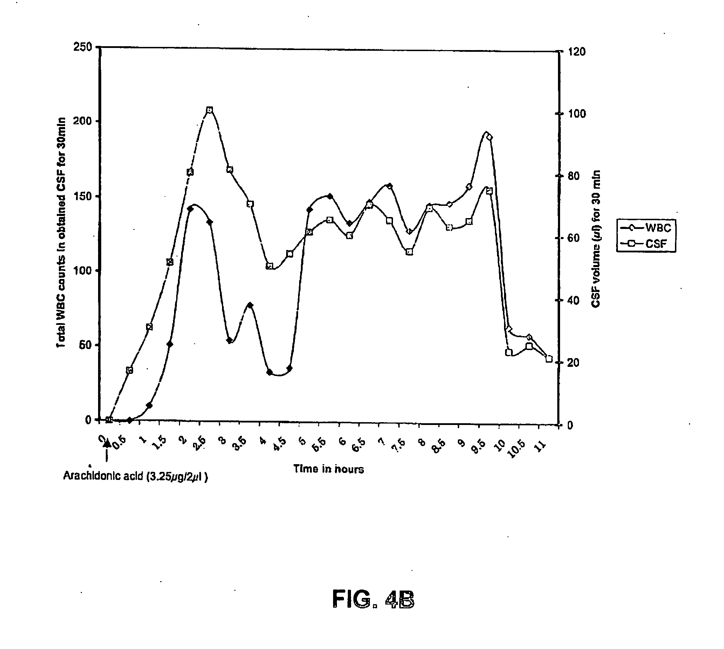

The volume of CSF increased immediately after the administration of arachidonic acid (3.25 µg/2µl) to the subarachnoid space. CSF volume peaked within 3.5 hours (Figs. 4A and Fig. 4B). It gradually decreased 4-5 hours after the AA administration, but it was seen to remain at a continuous level during the 11-hour observation period at this dose level.

Infiltration of WBC was not seen within the first 30 minutes, which may implicate an acute transient phase of brain inflammation, but then started to increase slowly (Fig. 4A and 4B). The changes of the total WBC counts and the volume of CSF rise and fall in parallel thereafter. This suggests a delayed subacute phase that is most characterized by the infiltration of leukocytes and phagocytic cells. Neither increased CSF nor WBC count was observed in the control animal. This was the normal state, as the pressure in the central nervous system is very low (6-10 mm Hg) and there are usually no WBCs in CSF.

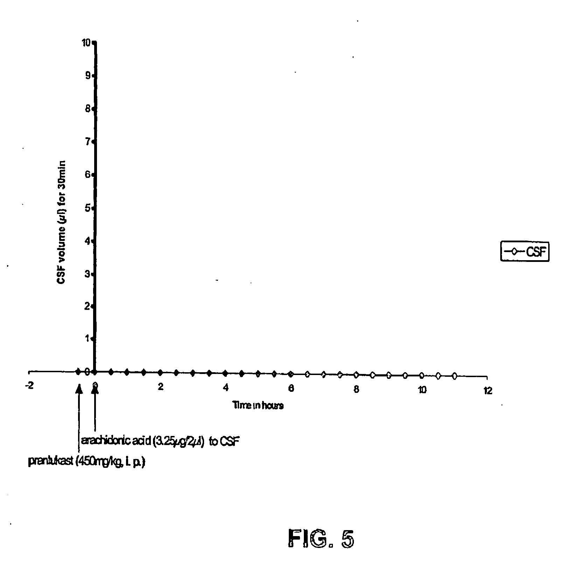

Pre-treatment using the leukotriene C4 and D4 receptor antagonist, pranlukast (490 mg/kg i.p.) thirty minutes before AA administration(3.25µg/2µl) completely inhibited the increase of CSF volume (n=4)(Fig. 5). This shows that pranlukast blocks an increase in the permeability of endothelial cells in the brain capillaries, as well as the infiltration of white blood cells.

### Example 3. Studies of the effects of the leukotriene receptor antagonist, pranlukast

### Evaluation of the increases of the permeability of endothelial cells in the brain capillaries and WBC infiltration to the CSF during inflammation

Using the above-described method, it is possible to measure the changes in permeability over time in the same animal. No other method has been able to measure inflammation over time using the same animals, and as a result, it has been necessary to sacrifice at least four animals (to attain statistical significance) at certain time periods. For example, if it is necessary to investigate the process of inflammation every thirty minutes for ten hours (n=4 for every thirty minutes), then 80 rats would need to be sacrificed. It is important to note that this method provides information not only regarding the changes in permeability of the brain capillaries, but also regarding changes in the infiltration of WBC into the CNS.

To obtain data on WBC infiltration into the CNS using this method, only four rats are required, avoiding the differences between individual animals for different time periods. Only this method can measure quantitatively both the permeability of the brain capillary and WBC infiltration into the CNS and can provide a more complete picture of the process of inflammation.

In the present study, the volume (µl) of cerebrospinal fluid (CSF) and white blood cell (WBC) counts in CSF was measured every thirty minutes for 11 hours from the same rat. One side of the cannula (PE10) was inserted into the subarachnoid space under pentobarbital anesthesia, avoiding neuronal damage (Fig. 3). If the brain tissue is damaged, the volume of CSF and WBC increases immediately without the administration of arachidonic acid. Since there are no WBC in the CSF in the normal rat, only rats that had no WBC in the CSF immediately after the cannulation were used. Another side of the cannula remained open and was used for the administration of nociceptive stimuli (arachidonic acid, LPS, dextran, etc.) and subsequently for collecting the CSF every thirty minutes during the experiment. The CSF volume was measured using a micropipette and WBC in the CSF were counted by means of a hemacytometer.

Arachidonic acid (3.2 µg/2µl) increased the CSF volume immediately after administration, where a maximum volume of 100-120 µl was reached within 3.5 hours. Although the volume decreased thereafter, the CSF volume of 60-80 µl was maintained for every thirty minutes during 4.0-5.0 hours post-administration. WBC could not be detected for the first thirty minutes after the administration of arachidonic acid, but then was observed to increase gradually. The increase or decrease of WBC was followed by changes in CSF volume over time.

Pranlukast (490 mg/kg) was administered intraperitoneally thirty minutes before the arachidonic acid application into the subarachnoid space. Pranlukast completely inhibited the increase in CSF volume and in the permeability of the brain capillary marked by WBC infiltration.

The method characterized the first and second inflammatory phases as mentioned above, an acute transient phase characterized by local vasodilation and increased capillary permeability (first phase) and a delayed, subacute phase, most prominently characterized by infiltration of leukocytes and phagocytic cells (second phase).

The brain capillaries have a relative absence of pinocytotic vesicles, a greatly increased number of mitochondria, and the presence of tight junctions in capillaries, unlike general capillaries which have clefts, fenestrae and prominent pinocytic vesicles. It is known that leukotriene receptor antagonists pass the BBB at very minimal levels, if at all. The findings in these studies suggest that leukotriene receptor antagonists peripherally inhibit the permeability of the endothelial cells in the brain capillary. The endothelial cells are closely juxtaposed to one another and form tight junctions. These endothelial cells on the "inside" brain capillaries are much less likely to leak compared to those on general capillaries, since general capillaries have cleft passages, fenestrae and prominent pinocytic vesicles.

The treatment of brain inflammation by the leukotriene C4 and D4 receptor antagonist might be due to peripheral inhibition of the permeability of the endothelial cells between the capillary lumen and the BBB. Therefore, this ability to decrease brain capillary permeability and to inhibit infiltration of WBC into the central nervous system may be more important therapeutically than the drug being able to pass through the BBB or to open the BBB to allow drugs to access the brain inflammation. Once open, the BBB permeability will increase and contribute to the formation of brain edema as the pressure of the brain capillary is higher than the intercranial pressure. Furthermore, infiltration of blood components to the central nervous system potentiates brain inflammation.

These methods provide new findings as follows:
(1) The inflammatory process (phase 1 and phase 2) can be measured quantitatively over time by these methods; and
(2) Pranlukast inhibits the second brain injury as well.

### Example 4: Effect of pranlukast and other anti-inflammatory drugs on models of traumatic brain injury in the rat

Pre-treatment of pranlukast has been found to result in longer survival in a rat ischemia model using a reversible 30 minute occlusion of both carotid arteries followed by reperfusion. One of 4-5 rats survived for one week when pranlukast (450 mg/kg i.p.) was administered 30 minutes before the occlusion.

Additionally, increased infiltration of WBC into the CSF caused by interleukin-6 were inhibited by pretreatment of pranlukast (administered at three different doses) in the intact dog.

It has been discovered by the present inventor that the leukotriene C4 and D4 antagonist, pranlukast (450 mg/kg. i.p.), completely inhibits inflammation caused by arachidonic acid (3.25 µg/2µl). This inflammation is the basis of increased permeability of the BBB and infiltration of WBC into the CSF. Therefore, the leukotriene C4 and D4 antagonist (pranlukast) is useful as a treatment of inflammation.

### Example 5: In vivo study in rats on inhibition of metastasis

In the experimental model used in this study, the volume of CSF, white blood cells and cancer cells that come out from the subarachnoid space through the canula are measured.
This experimental model serves as a new experimental model of metastasis.

To test for inhibition of metastasis, pranlukast is administered orally (instead of intraperitoneally as was done in Example 2) before intravenous injection of the cancer cells. Arachidonic acid (AA) is then administered to the subarachnoid space two hours after pranlukast administration. The volume of CSF, white blood cell counts and cancer cell counts in the CSF are measured every 30 minutes. The absence of cancer cells in the CSF demonstrates that pranlukast inhibits metastasis using this new experimental model of metastasis.

### MATERIAL AND METHODS

### Cells and culture conditions

Cultured human cancer cell lines, Colo 201 (colon cancer) and MKN74 (lung cancer), and a cultured rat cancer cell line RCN9 were purchased from Health Science Research Resources Bank (Rinku-minamihama 2-11, Sennan-shi, Osaka, 590-0535, Japan). Human lung cancer cell line QG56 was a gift from Dr. Yukito Ichinose, Department of Laboratory Medicine, Kyusyu Cancer Center, Hukuoka, Japan. Cells were cultured at 37°C and 5% CO₂, in RPMI-1640 (SIGMA, MO, USA) supplemented with 5% fetal bovine serum, 2mM L-glutamine (Gibco), 100 units penicillin, 100µg/ml streptomycin (Gibco Invitrogen Corp., Grand Island, N.Y., USA).

### Animals

RCN-9, a colon cancer cell line originating from Fisher 344 male rats (body weight 400±50g), and the Fisher 344 male rats were purchased from Sankyou Lab-Service Co.

### Chemicals

Sodium pentobarbital (Nembutal injection), a product of Abbott Laboratories (Abbott Park, IL, USA) and PKH67 Green Fluorescent Cell Linker Kit, a product of Zynaxis Cell Science Inc., and purchased from Dainippon Pharmaceutical Co. (Osaka, Japan). Arachidonic acid (sodium salt) from porcine liver was obtained from Calbiochem (San Diego, CA, USA). ONON, which contains 112.5mg/capsule of pranlukast, was purchased from Ono Pharmaceutical Co. (Osaka, Japan).

### Miscellaneous

Polyethylene tube (SP10, I.D. 0.28mm, O.D. 0.61mm) supplied from Natsume Seisakusyo Co., Sodium Heparin(5000 unit/5ml) was purchased from Shimizu Pharmaceutical Co.

### Morphological study

Cancer cells were stained with PKH linker kit. The morphological appearance of cancer cells using the fluorescence microscope (Axiovert S 100; Zeiss) was compared with that of the light microscope (Olympus CX21-22S-D) in the *in vitro* study. The magnification was 63 x 0.5 x 215/11.

Rats were anesthetized by sodium pentobarbital (50mg/kg i.p.). Whenever an animal recovered from the anesthesia (if the animal shows any kind of spontaneous movement), additional sodium pentobarbital was administered little by little to keep the deep anesthesia during the experimental period.

A femoral vein was exposed and a polyethylene tube filled with 0.002% heparin in saline was inserted. The other end of the polyethylene tube was connected to a three way cock valve for the intravenous injection of cancer cells. The animal was then placed in a stereotaxic unit (Type SRS-6 produced by Narishige Scientific Instrument Lab). Fur from the top of the head to the neck was shaved. The study area was disinfected with 70% ethanol. Skin and muscle over the cervical vertebrae were exposed. A hole was made using an 18Gxl 1/2 needle passing through the dura via the cisterna magna. One end (2-3mm) of an SP10 tube (5-6cm long) was inserted into the subarachnoid space. The cannula was fixed with cyanoacrylic glue to the peripheral tissue. The other end of the SP10 tube was used for administration of arachidonic acid (8.16ng/2µl of saline) and subsequently for collecting cerebrospinal fluid (CSF) every thirty minutes thereafter for 1-2 hours in the *in vivo* morphological study and for 8 hours in the interaction studies.

### Interaction study

This study below was designed to clarify whether or not pranlukast, a leukotriene C4 and D4 receptor antagonist, inhibits the extravasation of tumor cells from the capillary.

### Preparation

As a capsule of Onon contains 112.5 mg of pranlukast, the powder from two capsules was stirred in 2.295 ml of deionized water with the electric mixer immediately before oral administration. The volume of administration of pranlukast was 5ml/kg (dose = 490mg/kg).

The number of cancer cells were prepared to 1/100 of the number of neutrophils in each rat using PBS. Because of body weight (400±50g), the number of cancer cells were between 1.88 x 10⁴ and 2.43 x 10⁴ and the volume of administration of cancer cell was adjusted to 1ml for each rat.

Arachidonic acid was dissolved in saline and 816.25ng/2µl was administered for each rat.

Three different treatments were administered as follows;

1^{st} treatment was pranlukast (490mg/kg p.o.) or deionized water(p.o.).

2^{nd} treatment was with cancer cells or PBS

3^{rd} treatment was with arachidonic acid or saline.

The first treatment was administered 2 hours before arachidonic acid. The second treatment (cancer cells i.v.) was administered 2-3 minutes before arachidonic acid. Two canula in each animal to the femoral vein and to the subarachnoid space were inserted between the 1^{st} treatment and 2^{nd} treatment.

The experimental schedule is as shown in Table 1 below. The number on the right side of table corresponds to that of the figures for the results. Four rats were used for each group.

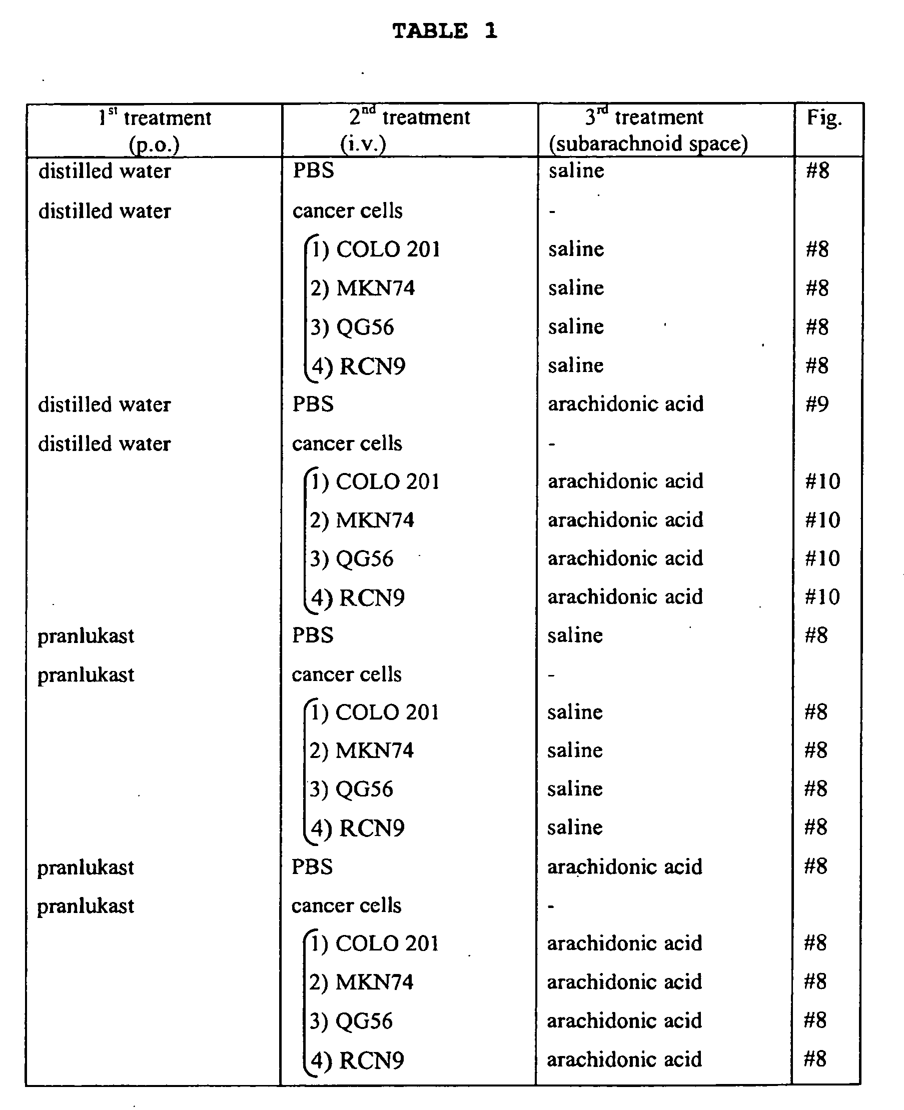

### Measurement

The volume (µl) of CSF, WBC counts, and cancer cells counts was measured after the 3^{rd} treatment for 8 hours.

### Statistics

The area under the time action curve for eight hours after arachidonic acid were calculated for each animal and analyzed using a nonparametric test, the Mann-Whiteney U-test for the inhibitory effect of pranlukast (490mg/kg) on tumor cell counts, white blood cell counts, and CSF since pranlukast (490mg/kg) inhibits completely (the area was zero). To analyze whether or not human cancer cells, as foreign substances, affect the immune system in rats, each parameter, i.e., white blood cells counts, tumor cells counts and CSF volume with rat cancer cells (RCN9) was compared with the corresponding parameters of COLO201, MKN74, and QG56, respectively, using area under time action curves and analysis of variance (Dunnet). Probability value was p<0.05 to be significant.

### RESULTS

### Morphological study

In the surface of a central capillary in the brain, there are many ditches that serve as the blood brain barrier (Fig. 6). White blood cells, as well as cancer cells, were able to come out from these openings of the blood brain barrier.

In the *in vitro* study, chromosomes can be seen through the cell membrane in all cancer cells, i.e., Colo201, MKN74, QG56, and RCN9. However, after the administration of arachidonic acid in the *in vivo* study, those cells did not show any chromosomes after the migration from the capillary to the tissue (in this case CSF). The shapes of cancer cells during extravasation changed from spherical to flat square (Figs. 7A-7C). Cancer cells migrated immediately after arachidonic acid treatment, followed by migration of white blood cells. White blood cells were also observed to have a square shape at their adhesion in the capillary but not after the transendothelial migration (Figs. 7A-7C). As shown in Fig. 7C, it appears that cancer cells adhere to CAM on endothelial cells before starting their transendothelial migration. This phenomenon is likely the same with cancer cells as with white blood cell (neutrophils) as seen by the square shape of the neutrophils adhering to CAM or the endothelial cells (Figs. 7A and 7B). There are four adhering spots giving the appearance of a square shape.

### Interaction study

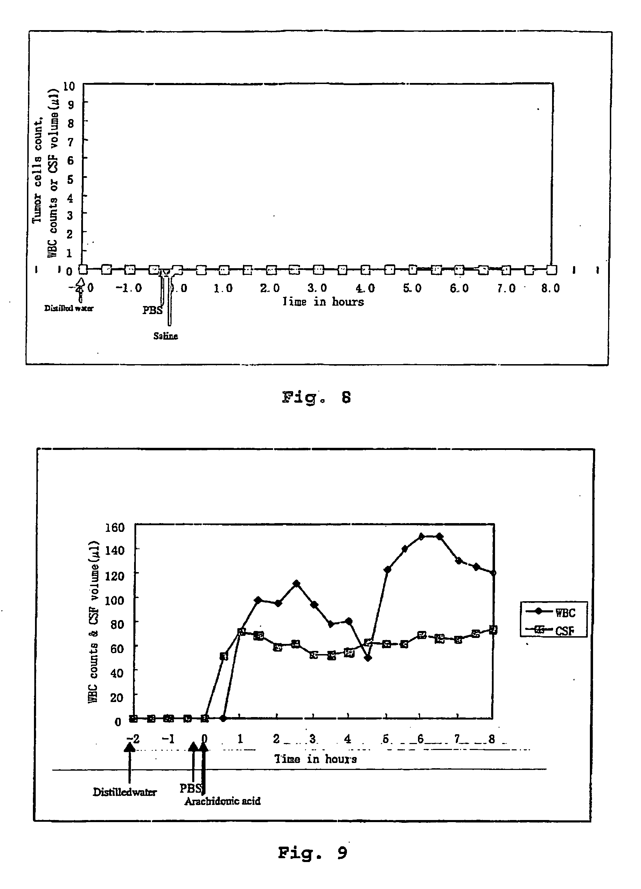

Neither distilled water, PBS nor saline (Distilled water-PBS-saline) had any effect (Figure 8). Although cancer cells were present in the blood vessel, no increase in capillary permeability or in the migration of white blood cells or cancer cells occurred without treatment with arachidonic acid, i.e., Colo201 (distilled water-Colo201-saline), MKN74 (distilled water-MKN74-saline), QG56 (distilled water-QG56-saline) and RCN9(distilled water-RCN9-saline) in which the cancer cells were administered in place of PBS, but had the same response as PBS (no effect) shown in Fig. 8.

Capillary permeability increased immediately after arachidonic acid and the extravasation of white blood cells started 30 minutes after arachidonic acid treatment (distilled water-PBS-arachidonic acid; Figure 9). These phenomena were seen during 8 hour observation periods, and the areas under the time action curves were statistically significant compared to capillary permeability with the control (distilled water-PBS-saline).

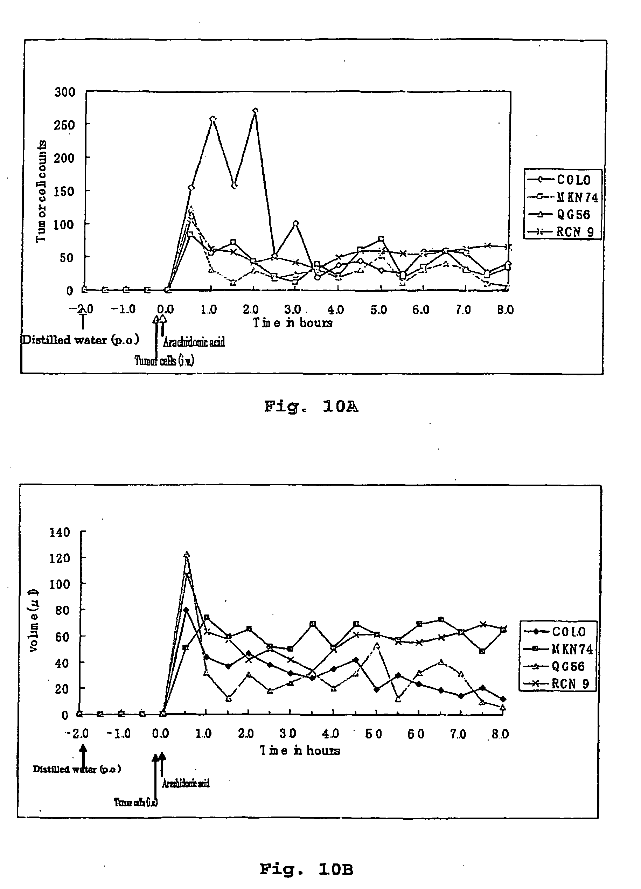

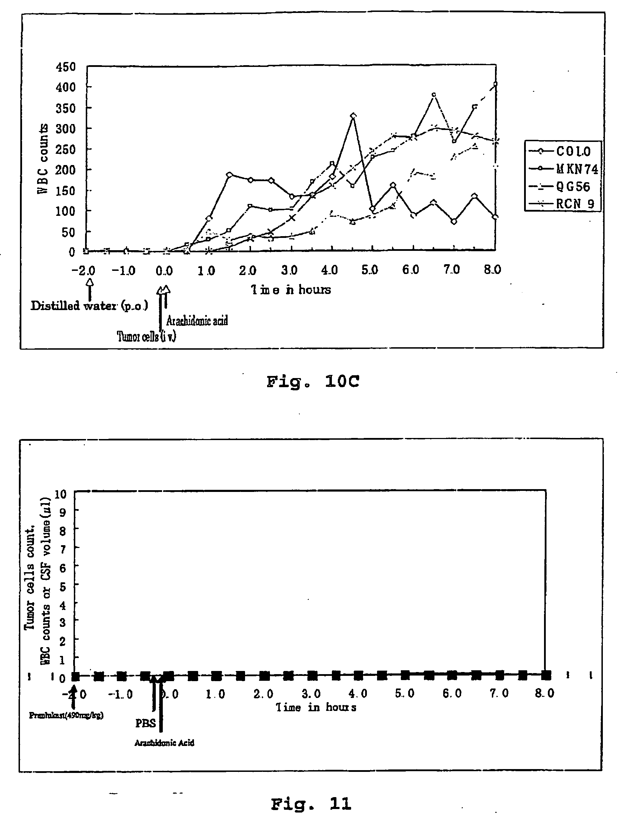

The administration of distilled water-Colo201-arachidonic acid; distilled water-MKN74-arachidonic acid; distilledwater-QG56-arachidonic acid; and distilled water-RCN9-arachidonic acid induced an increase in the extravasation of tumor cells (Figure 10A), capillary permeability (Figure 10B), and white blood cells (Figure 10C).

There was no effect with pranlukast by itself. The graph is the same as Fig. 8, except that pranlukast replaced distilled water. Pranlukast also did not have any effect (same response as in the graph of Fig. 8) when cancer cells were present in the blood vessel without arachidonic acid, i.e., pranlukast-Colo201-saline, pranlukast-MKN74-saline, pranlukast-QG56-saline, and pranlukast-RCN9-saline.

However, the administration of pranlukast inhibited the increased capillary permeability and the extravasation of white blood cells caused by the administration of arachidonic acid (Figure 11). Furthermore, when tumor cells were present, pranlukast significantly inhibited not only the increasing capillary permeability but also the extravasation of tumor cells upon administration of arachidonic acid, i.e., Colo201 (pranlukast-Colo201-arachidonic acid), MKN74 (pranlukast-MKN74-arachidonic acid) QG56 (pranlukast-QG56-arachidonic acid) and RCN9 (pranlukast-RCN9-arachidonic acid), which all show the response as in Fig. 11.

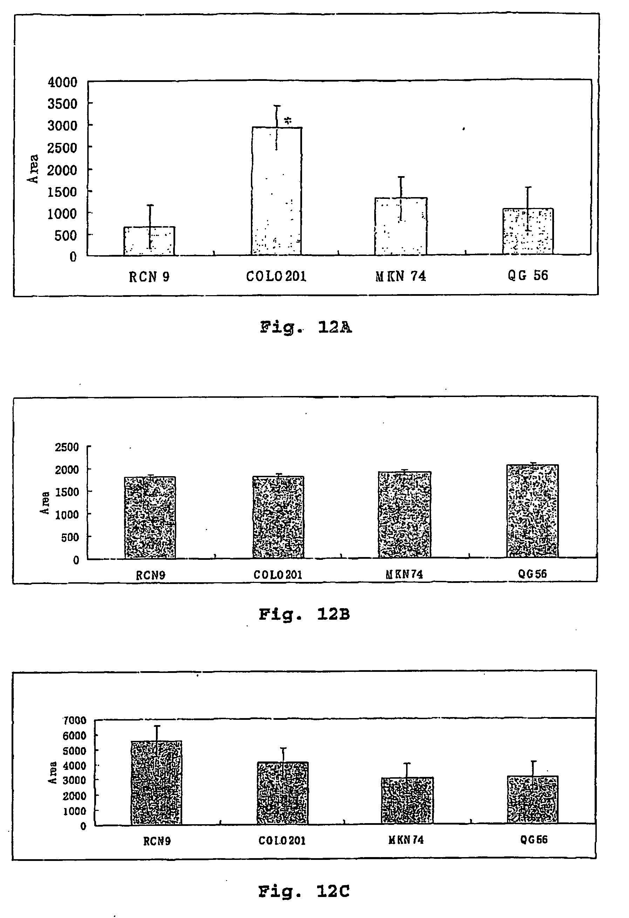

Three kinds of human cancer cells were analyzed for their effect on the rat immune systems as foreign substances. The results of RCN9 on the extravasation of tumor cell (Figure 12A), CSF volume (Figure 12B), and WBC counts (Figure 12C) were analyzed and compared with the results from Colo201 (distilled water-Colo201-arachidonic acid), MKN74 (distilled water-MKN74-arachidonic acid) and QG56 (distilled water- QG56- arachidonic acid). The transendothelial migration of Colo201 cancer cells, but not the other two types of cancer cells, was found to be significantly increased compared to that of RCN9 (Figure 12A). However, the increased capillary permeability and white blood cell counts obtained with RCN9 (distilled water-RCN9-arachidonic acid) was not observed to be significantly different those obtained with Colo201 (distilled water-Colo201-arachidonic acid), MKN74 (distilled water-MKN74-arachidonic acid) and QG56 (distilled water-QG56 -arachidonic acid) respectively (Figures 12B and 12C). In the case of MKN74 (distilled water-MKN74-arachidonic acid), extravasation of white blood cells was seen earlier (within 30 minutes after arachidonic acid; Figure 10C) than extravasation of white blood cells without cancer cells (distilled water-PBS-arachidonic acid; Figure 9).

### DISCUSSION

Pranlukast, a leukotriene C4 and D4 receptor antagonist that does not cross the blood brain barrier, inhibits capillary permeability and extravasation of white blood cells. This suggests that the mode of action of pranlukast is peripheral and from the interior of the blood vessel including the endothelial cells and the capillary rumen.

Morphological studies suggest that both tumor cells and white blood cells migrate through the openings of the blood brain barrier in the central capillary as well as through the openings between endothelial cells in the peripheral capillary.

The ability of cancer cells to change their shape, such as to being thin and square in shape, is beneficial to their migration from the capillary to the tissue. Thin shapes cancer cells can easily pass through an opening even if the opening is really narrow because the square corner of the flattened cancer cell is advantageous in starting the transendothelial migration through the narrow opening.

The principle of the inhibition of tumor cell migration by pranlukast may be due to keeping the openings closed. In fact, the observations in Figs. 7A and 7B suggest that cancer cells start to migrate sooner than white blood cells that still adhere to the endothelial cells. The starting time for the extravasation of white blood cells to begin might be affected by the presence of some tumor cells, such as MKN74 in the present study (Fig. 10C).

In the present study, three different kinds of human cancer cells and one kind rat cancer cell were used. Extravasation from the capillary of all four kinds of different cancer cells were observed, where the counts of the human cancer cell Colo201 at 8 hours(=area) increased significantly, whereas the counts of the other two kinds of human cancer cell, i.e., MKN74 and QG56, were not significant different from RCN9.

Increased capillary permeability and increased white blood cell counts obtained by rat cancer cells (RCN9) were not significantly different when compared to capillary permeability and white blood cell counts obtained with the other three kinds of human tumor cells. This suggests that human cancer cells as foreign substances might have only a small effect on the rat immune system. However, regarding the shortened start time interval for extravasation of white blood cells in the presence of MKN74 (distilled water-MKN74-arachidonic acid; Figure 10C) when compared to the start time in the absence of MKN74 (distilled water-PBS-arachidonic acid), the increased capillary permeability immediately after treatment with arachidonic acid and the start of extravasation of white blood cells 30 minutes after arachidonic acid Fig. 9, suggest some effect on the rat immune system. This shortened time interval might be affected due to extravasation of MKN74 that might have made the openings in the blood brain barrier large enough to start the transendothelial migration of white blood cells. However, the total effect as measured by the area under time action curve for 8 hours were analyzed and not found to be statistically significant (Figure 12C).

The insertion of a canula into a vein for the administration of tumor cells into the blood vessel or into the subarachnoid space for obtaining specimens such as tumor cells that have migrated from the central capillary provides a means for studying hepatic tumor cell metastasis. As far as known, there is no site such as the subarachnoid space where one can count the white blood cells or tumor cells that have migrated from a capillary.

It is known that the surface of both cancer cells and white blood cells is covered with Sialyl Lewis, a glycoprotein that differentiates between species and is the ligand for the p-selectin and e-selectin on endothelial cells. Because Sialyl Lewis in human cancer cells may not recognize these selectins on the endothelial cell, it is believed that the human cancer cells may not adhere to the endothelial cells, and would therefore be hard for the cancer cells to migrate across the capillary.

In the present study, transendothelial migration was observed for all three human cancer cells following treatment with arachidonic acid and was inhibited by pretreatment with pranlukast in the rat. Therefore, one can study human tumor cell metasatasis *in vivo* in rats.

In conclusion, the present inventor found that:
1) Pranlukast inhibits the extravasation of tumor cells and white blood cells due to inhibition of capillary permeability.
2) Pranlukast can be used as anti-tumor metastasis agent/drug because of its safe and prolonged action.
3) Inhibiting increased capillary permeability is essential for an anti-cancer metastasis.
4) As human cancer cells can migrate from the rat capillary to the tissue, this animal model can be used as an assay for the anti-cancer metastasis property of a drug.

### REFERENCES

Bevilacqua, M. P. and Nelson, R. M., J. Clin. Invest. 91 379-387 (1993)
Bevilacqua, M. P. et al., Science 243: 1160-1165 (1989)
Brandley, B, et al., Cell 63, 861-863 (1990)
Johnston et al., Cell 56: 1033-1044 (1989)
Lasky et al, Cell 56: 1045-1055 (1989)
Piper, Formation and actions of leukotrienes, Physiol. Rev., 64(2):744-61 (1984)
Sialy Lewis, Dependent Alterations in Leukocyte Kinetics In Vivo, Circulation Research, 77(5):879-887 (1995)
Siegalman, M. et al., Science 243: 1165-1172 (1989)
Springer, T. and Lasky, L. A., Nature 349, 19-197 (1991)
Stoolman, L. M., Cell 56: 907-910 (1989)
Takeda et al., Contribution of Carbohydrate Antigens Sialyl Lewis A and Sialyl Lewis X to Adhesion of Human Cancer Cells to Vascular Endotherlium, Cancer Research, 53:354-361 (Jan. 15, 1993)
Tedder et al., J. Exp. Med. 170: 123-133 (1989)
von Sprecher et al., Strategies in the design of peptidoleukotriene antagonists, J. Limpid Mediat. 6:265-273(1993)
Wilkinson, G. R. Pharmacokinetics. In, Goodman and Gilman's The pharmacological Basis of Therapeutics. 10th ed. (2001)
Zeng et al., Protective Effect of ONO-1078, A Leukotriene. Antagonis on Foca Cerebral Ischemia in Mice, Acta. Pharamaceutics Sinica, 36(2):148-150 (2001)
Zhang, W., et al., Neuroprotective Effect of ONO-1078, A Leukotriene Receptor Antagonist, on Focal Cerebral Ischemia in Rats, Acta Pharmacol Sin, 23(10):871-877 (2002)

## Claims

1. Use of a leukotriene C4 and D4 receptor antagonist, or a pharmaceutically-acceptable salt thereof, for the preparation or manufacture of a medicament for inhibiting tumor metastasis in the absence of any other compounds having anti-tumor cell adhesion, anti-invasion or anti-metastasis properties.

2. Use of a leukotriene C4 and D4 receptor antagonist, or a pharmaceutically-acceptable salt thereof, for the preparation or manufacture of a medicament for inhibiting tumor metastasis, comprising administering an effective amount of a leukotriene C4 and D4 receptor antagonist, or
a pharmaceutically acceptable salt thereof, to a subject in need thereof who is not concurrently undergoing a surgical procedure.

3. Use of a leukotriene C4 and D4 receptor antagonist, or a pharmaceutically-acceptable salt thereof, for the preparation or manufacture of a medicament for inhibiting tumor cell adhesion to endothelial cells and/or inhibiting capillary permeability in the form of transendothelial migration of tumor cells, comprising causing an effective amount of a leukotriene C4 and D4 receptor antagonist, or pharmaceutically acceptable salt thereof, to contact endothelial cells to inhibit capillary permeability and/or tumor cell adhesion to the endothelial cells contacted with the leukotriene C4 and D4 receptor receptor antagonist.

4. Use according to claim 2 or 3, wherein the leukotriene C4 and D4 receptor antagonist, or a pharmaceutically acceptable salt thereof, is administered orally.

5. Use according to claim 1, 2 or 3, wherein the leukotriene C4 and D4
receptor antagonist is a compound of formula (I) 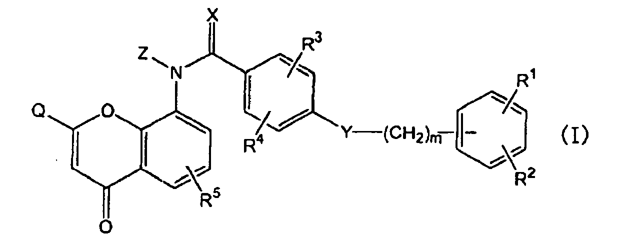 wherein:
R¹, R², R³, R⁴ and R⁵ are the same or different and is hydrogen, halogen, hydroxyl, amino, carboxy, nitro, cyano, lower alkyl,
lower alkoxy, lower alkanoyl, lower alkanoyloxy, lower alkoxycarbonyl, mono or di lower alkyl substituted-amino, aralkyl, aryl, or heteroaryl;
Q is carboxyl, lower alkoxycarbonyl, or tetrazoyl;
X is oxygen, sulfur, or -NR- (wherein R is hydrogen or lower alkyl);
Z is hydrogen or lower alkyl; and
m is an integer from 1 to 6.

6. Use according to claim 5, wherein the leukotriene C4 and D4 receptor antagonist is pranlukast..

7. A leukotriene C4 and D4 receptor antagonist, or a pharmaceutically-acceptable salt thereof, for use in inhibiting tumor metastasis in the absence of any other compounds having anti-tumor cell adhesion, anti-invasion or anti-metastasis properties.

8. A leukotriene C4 and D4 receptor antagonist, or a pharmaceutically-acceptable salt thereof, for use in inhibiting tumor metastasis, comprising administering an effective amount of a leukotriene C4 and D4 receptor antagonist, or a pharmaceutically acceptable salt thereof, to a subject in need thereof who is not concurrently undergoing a surgical procedure.

9. A leukotriene C4 and D4 receptor antagonist, or a pharmaceutically-acceptable salt thereof for inhibiting tumor cell adhesion to endothelial
cells and/or inhibiting capillary permeability in the form of transendothelial migration of tumor cells, comprising causing an effective amount of a leukotriene C4 and D4 receptor antagonist, or pharmaceutically acceptable salt thereof, to contact endothelial cells to inhibit capillary permeability and/or tumor cell adhesion to the endothelial cells contacted with the leukotriene C4 and D4 receptor receptor antagonist.

10. A leukotriene C4 and D4 receptor antagonist, or a pharmaceutically-acceptable salt thereof according to claims 8 or 9, wherein the leukotriene C4 and D4 receptor antagonist, or a pharmaceutically acceptable salt thereof, is administered orally.

11. A leukotriene C4 and D4 receptor antagonist, or a pharmaceutically-acceptable salt thereof according to claims 7, 8 or 9, wherein the leukotriene C4 and D4 receptor antagonist is a compound of formula (I) 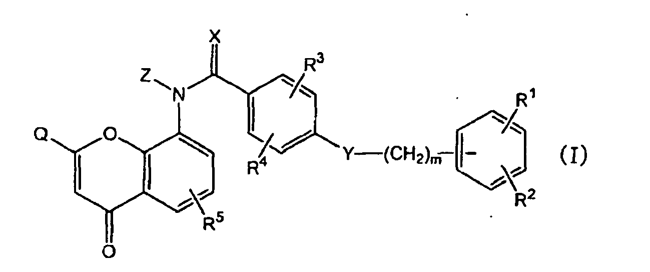 wherein:
R¹, R², R³, R⁴ and R⁵ are the same or different and is hydrogen, halogen, hydroxyl, amino, carboxy, nitro, cyano, lower alkyl,
lower alkoxy, lower alkanoyl, lower alkanoyloxy, lower alkoxycarbonyl, mono or di lower alkyl substituted-amino, aralkyl, aryl, or heteroaryl;
Q is carboxyl, lower alkoxycarbonyl, or tetrazoyl;
X is oxygen, sulfur, or -NR- (wherein R is hydrogen or lower alkyl);
Z is hydrogen or lower alkyl; and
m is an integer from 1 to 6.

12. A leukotriene C4 and D4 receptor antagonist, or a pharmaceutically-acceptable salt thereof according to claim 11, wherein the leukotriene C4 and D4 receptor antagonist is pranlukast.

## Patentansprüche

1. Verwendung eines Leukotrien-C4 und D4-Rezeptorantagonisten oder eines pharmazeutisch akzeptablen Salzes davon für die Zubereitung oder Herstellung eines Medikaments zum Hemmen von Tumormetastasierung bei Fehlen von jeglichen anderen Verbindungen mit Eigenschaften gegen Tumorzelladhäsion, gegen Invasion oder gegen Metastasierung.

2. Verwendung eines Leukotrien-C4 und D4-Rezeptorantagonisten oder eines pharmazeutisch akzeptablen Salzes davon für die Zubereitung oder Herstellung eines Medikaments zum Hemmen von Tumormetastasierung, umfassend das Verabreichen einer wirksamen Menge eines Leukotrien-C4 und D4-Rezeptorantagonisten oder eines pharmazeutisch akzeptablen Salzes davon an einen Probanden, der dies benötigt und sich nicht gleichzeitig einem chirurgischen Verfahren unterzieht.

3. Verwendung eines Leukotrien-C4 und D4-Rezeptorantagonisten oder eines pharmazeutisch akzeptablen Salzes davon für die Zubereitung oder Herstellung eines Medikaments zum Hemmen von Tumorzelladhäsion an Endothelzellen und/oder zum Hemmen der Kapillarpermeabilität in Form von transendothelialer Migration von Tumorzellen, umfassend das Herbeiführen eines Kontakts von einer wirksamen Menge eines Leukotrien-C4 und D4-Rezeptorantagonisten oder eines pharmazeutisch akzeptablen Salzes davon mit Endothelzellen, um die Kapillarpermeabilität und/oder die Tumorzelladhäsion an Endothelzellen, die mit dem Leukotrien-C4 und D4-Rezeptorantagonisten in Kontakt gebracht wurden, zu hemmen.

4. Verwendung gemäß Anspruch 2 oder 3, wobei der Leukotrien-C4 und D4-Rezeptorantagonist oder ein pharmazeutisch akzeptables Salz davon oral verabreicht wird.

5. Verwendung gemäß Anspruch 1, 2 oder 3, wobei der Leukotrien-C4 und D4-Rezeptorantagonist eine Verbindung mit der Formel (I) ist, 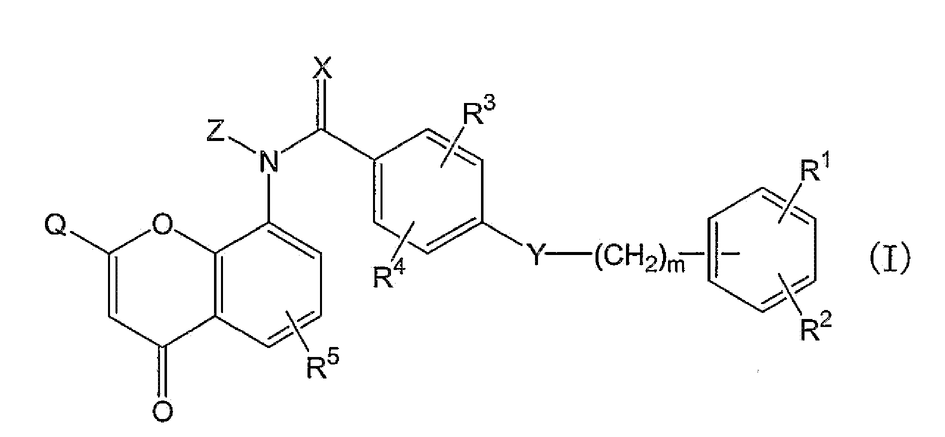 wobei:
R¹, R², R³, R⁴ und R⁵ gleich oder verschieden sind und Wasserstoff, Halogen, Hydroxyl, Amino, Carboxy, Nitro, Cyano, Niederalkyl, Niederalkoxy, Niederalkanoyl, Niederalkanoyloxy, Niederalkoxycarbonyl, mono- oder di-niederalkylsubstituiertes Amino, Aralkyl, Aryl oder Heteroaryl sind;
Q Carboxyl, Niederalkoxycarbonyl oder Tetrazoyl ist;
X Sauerstoff, Schwefel oder -NR- (wobei R Wasserstoff oder Niederalkyl ist) ist;
Z Wasserstoff oder Niederalkyl ist und
m eine Ganzzahl von 1 bis 6 ist.

6. Verwendung nach Anspruch 5, wobei es sich bei dem Leukotrien-C4 und D4-Rezeptorantagonisten um Pranlukast handelt.

7. Leukotrien-C4 und D4-Rezeptorantagonist oder pharmazeutisch akzeptables Salz davon zur Verwendung beim Hemmen von Tumormetastasierung bei Fehlen von jeglichen anderen Verbindungen mit Eigenschaften gegen Tumorzelladhäsion, gegen Invasion oder gegen Metastasierung.

8. Leukotrien-C4 und D4-Rezeptorantagonist oder pharmazeutisch akzeptables Salz davon zur Verwendung beim Hemmen von Tumormetastasierung, umfassend das Verabreichen einer wirksamen Menge eines Leukotrien-C4 und D4-Rezeptorantagonisten oder eines pharmazeutisch akzeptablen Salzes davon an einen Probanden, der dies benötigt und sich nicht gleichzeitig einem chirurgischen Verfahren unterzieht.

9. Leukotrien-C4 und D4-Rezeptorantagonist oder pharmazeutisch akzeptables Salz davon zum Hemmen von Tumorzelladhäsion an Endothelzellen und/oder zum Hemmen der Kapillarpermeabilität in Form von transendothelialer Migration von Tumorzellen, umfassend das Herbeiführen eines Kontakts von einer wirksamen Menge eines Leukotrien-C4 und D4-Rezeptorantagonisten oder eines pharmazeutisch akzeptablen Salzes davon mit Endothelzellen, um die Kapillarpermeabilität und/oder die Tumorzelladhäsion an Endothelzellen, die mit dem Leukotrien-C4 und D4-Rezeptorantagonisten in Kontakt gebracht wurden, zu hemmen.

10. Leukotrien-C4 und D4-Rezeptorantagonist oder pharmazeutisch akzeptables Salz davon gemäß den Ansprüchen 8 oder 9, wobei der Leukotrien-C4 und D4-Rezeptorantagonist oder ein pharmazeutisch akzeptables Salz davon oral verabreicht wird.

11. Leukotrien-C4 und D4-Rezeptorantagonist oder pharmazeutisch akzeptables Salz davon gemäß den Ansprüchen 7, 8 oder 9, wobei der Leukotrien-C4 und D4-Rezeptorantagonist der Leukotrien-C4 und D4-Rezeptorantagonist eine Verbindung mit der Formel (I) ist, 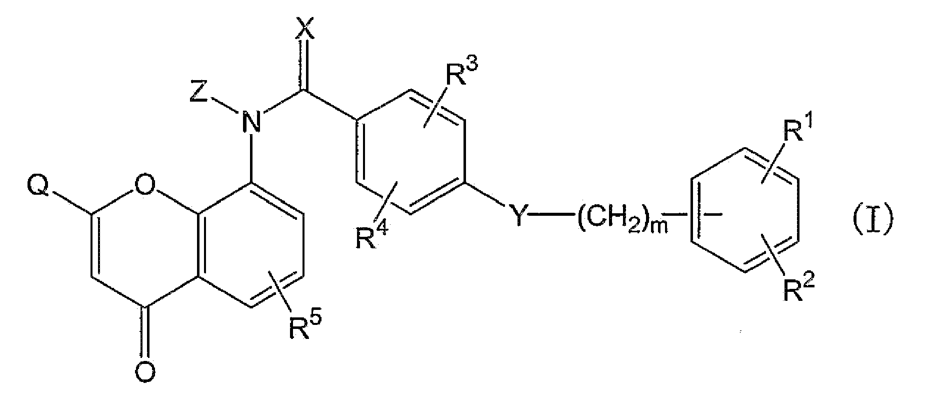 wobei:
R¹, R², R³, R⁴ und R⁵ gleich oder verschieden sind und Wasserstoff, Halogen, Hydroxyl, Amino, Carboxy, Nitro, Cyano, Niederalkyl, Niederalkoxy, Niederalkanoyl, Niederalkanoyloxy, Niederalkoxycarbonyl, mono- oder di-niederalkylsubstituiertes Amino, Aralkyl, Aryl oder Heteroaryl sind;
Q Carboxyl, Niederalkoxycarbonyl oder Tetrazoyl ist;
X Sauerstoff, Schwefel oder -NR- (wobei R Wasserstoff oder Niederalkyl ist) ist;
Z Wasserstoff oder Niederalkyl ist und
m eine Ganzzahl von 1 bis 6 ist.

12. Leukotrien-C4 und D4-Rezeptorantagonist oder pharmazeutisch akzeptables Salz davon gemäß Anspruch 11, wobei es sich bei dem Leukotrien-C4 und D4-Rezeptorantagonisten um Pranlukast handelt.

## Revendications

1. Utilisation d'un antagoniste de récepteur de leucotriène C4 et D4, ou d'un sel pharmaceutiquement acceptable de celui-ci, pour la préparation ou la fabrication d'un médicament destiné à inhiber la métastase de tumeur en l'absence de tout autre composé ayant des propriétés anti-adhérence de cellules tumorales, anti-invasion ou anti-métastase.

2. Utilisation d'un antagoniste de récepteur de leucotriène C4 et D4, ou d'un sel pharmaceutiquement acceptable de celui-ci, pour la préparation ou la fabrication d'un médicament destiné à inhiber la métastase de tumeur, comprenant l'administration d'une quantité efficace d'un antagoniste de récepteur de leucotriène C4 et D4, ou d'un sel pharmaceutiquement acceptable de celui-ci, à un sujet qui en a besoin et qui ne subit pas simultanément une procédure chirurgicale.

3. Utilisation d'un antagoniste de récepteur de leucotriène C4 et D4, ou d'un sel pharmaceutiquement acceptable de celui-ci, pour la préparation ou la fabrication d'un médicament destiné à inhiber l'adhérence de cellules tumorales à des cellules endothéliales et/ou à inhiber la perméabilité capillaire sous la forme d'une migration transendothéliale de cellules tumorales, comprenant le fait d'amener une quantité efficace d'un antagoniste de récepteur de leucotriène C4 et D4, ou d'un sel pharmaceutiquement acceptable de celui-ci, à venir en contact avec les cellules endothéliales pour inhiber la perméabilité capillaire et/ou l'adhérence de cellules tumorales aux cellules endothéliales mises en contact avec l'antagoniste de récepteur de leucotriène C4 et D4.

4. Utilisation selon la revendication 2 ou 3, dans laquelle l'antagoniste de récepteur de leucotriène C4 et D4, ou un sel pharmaceutiquement acceptable de celui-ci, est administré par voie orale.

5. Utilisation selon la revendication 1, 2 ou 3, dans laquelle l'antagoniste de récepteur de leucotriène C4 et D4 est un composé de formule (I) 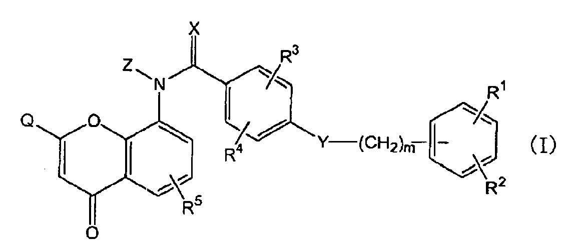 dans laquelle :
R¹, R², R³, R⁴ et R⁵ sont identiques ou différents et sont un atome d'hydrogène, d'halogène, un groupe hydroxyle, amino, carboxy, nitro, cyano, alkyle inférieur, alcoxy inférieur,
alcanoyle inférieur, alcanoyloxy inférieur, alcoxycarbonyle inférieur, amino mono- ou di-substitué par alkyle inférieur, aralkyle, aryle ou hétéroaryle ;
Q est un groupe carboxyle, alcoxycarbonyle inférieur ou
tétrazoyle ;
X est un atome d'oxygène, de soufre, ou -NR- (où R est un atome d'hydrogène ou un groupe alkyle inférieur) ;
Z est un atome d'hydrogène ou un groupe alkyle inférieur ; et
m est un nombre entier de 1 à 6.

6. Utilisation selon la revendication 5, dans laquelle l'antagoniste de récepteur de leucotriène C4 et D4 est le pranlukast.

7. Antagoniste de récepteur de leucotriène C4 et D4, ou sel pharmaceutiquement acceptable de celui-ci, à utiliser dans l'inhibition de la métastase de tumeur en l'absence de tout autre composé ayant des propriétés anti-adhérence de cellules tumorales, anti-invasion ou anti-métastase.

8. Antagoniste de récepteur de leucotriène C4 et D4, ou sel pharmaceutiquement acceptable de celui-ci, à utiliser dans l'inhibition de la métastase de tumeur, comprenant l'administration d'une quantité efficace d'un antagoniste de récepteur de leucotriène C4 et D4, ou d'un sel pharmaceutiquement acceptable de celui-ci à un sujet qui en a besoin et qui ne subit pas simultanément une procédure chirurgicale.

9. Antagoniste de récepteur de leucotriène C4 et D4, ou sel pharmaceutiquement acceptable de celui-ci, pour inhiber l'adhérence de cellules tumorales à des cellules endothéliales et/ou inhiber la perméabilité capillaire sous la forme d'une migration transendothéliale de cellules tumorales, comprenant le fait d'amener une quantité efficace d'un antagoniste de récepteur de leucotriène C4 et D4, ou d'un sel pharmaceutiquement acceptable de celui-ci, à venir en contact avec les cellules endothéliales pour inhiber la perméabilité capillaire et/ou l'adhérence de cellules tumorales aux cellules endothéliales mises en contact avec l'antagoniste de récepteur de leucotriène C4 et D4.

10. Antagoniste de récepteur de leucotriène C4 et D4, ou sel pharmaceutiquement acceptable de celui-ci selon la revendication 8 ou 9, dans lequel l'antagoniste de récepteur de leucotriène C4 et D4, ou un sel pharmaceutiquement acceptable de celui-ci est administré par voie orale.

11. Antagoniste de récepteur de leucotriène C4 et D4, ou sel pharmaceutiquement acceptable de celui-ci selon la revendication 7, 8 ou 9, dans lequel l'antagoniste de récepteur de leucotriène C4 et D4 est un composé de formule (I) 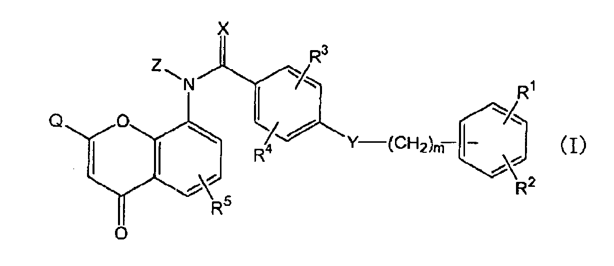 dans laquelle :
R¹, R², R³, R⁴ et R⁵ sont identiques ou différents et sont un atome d'hydrogène, d'halogène, un groupe hydroxyle, amino, carboxy, nitro, cyano, alkyle inférieur, alcoxy inférieur,
alcanoyle inférieur, alcanoyloxy inférieur, alcoxycarbonyle inférieur, amino mono- ou di-substitué par alkyle inférieur, aralkyle, aryle ou hétéroaryle ;
Q est un groupe carboxy, alcoxycarbonyle inférieur ou tétrazoyle ;
X est un atome d'oxygène, de soufre, ou -NR- (où R est un atome d'hydrogène ou un groupe alkyle inférieur) ;
Z est un atome d'hydrogène ou un groupe alkyle inférieur ; et
m est un nombre entier de 1 à 6.

12. Antagoniste de récepteur de leucotriène C4 et D4, ou sel pharmaceutiquement acceptable de celui-ci selon la revendication 11, dans lequel l'antagoniste de récepteur de leucotriène C4 et D4 est le pranlukast.
